# EUROPEAN PATENT APPLICATION

(11) **EP 4 266 155 A1**
(43) Date of publication of application: **25.10.2023**
(21) Application number: 21922593.5
(22) Date of filing: 16.12.2021
(51) Int. Cl.: G06F 3/01

(54) **SKIN CARE CHECK-IN METHOD AND ELECTRONIC DEVICE**

(30) Priority: 28.01.2021 CN 202110116954
(71) Applicant: Huawei Technologies Co., Ltd., Shenzhen, Guangdong 518129 (CN)
(72) Inventor: XU, Zhe, Shenzhen, Guangdong 518129 (CN); LU, Yuewan, Shenzhen, Guangdong 518129 (CN)
(74) Representative: Epping - Hermann - Fischer
(86) International application number: PCT/CN2021/138745
(87) International publication number: WO 2022/161003

(57) **Abstract**

This application provides a skin care check-in method and an electronic device, and relates to the field of terminal technologies. The method includes: receiving a first operation that is input by a user; in response to the first operation, displaying at least one skin care check-in plan in which the user participates; obtaining first target information, where the first target information includes one or more of time, a location of the electronic device, a movement status of the user, and a schedule of the electronic device; determining, based on the first target information, whether a preset implementation condition for implementing the at least one skin care check-in plan is met; and if a determining result is that the preset implementation condition is met, displaying first reminder information, where the first reminder information is used to prompt the user to implement the at least one skin care check-in plan. In this method, check-in for a skin care behavior of a user can be automatically performed, thereby reducing a workload of the user and improving user experience.

## Description

This application claims priority to Chinese Patent Application No. 202110116954.9, filed with the China National Intellectual Property Administration on January 28, 2021 and entitled "SKIN CARE CHECK-IN METHOD AND ELECTRONIC DEVICE", which is incorporated herein by reference in its entirety.

### TECHNICAL FIELD

This application relates to the field of terminal technologies, and in particular, to a skin care check-in method and an electronic device.

### BACKGROUND

Currently, users have an urgent need for a skin care method. To help the users develop facial care habits and provide guidance for the users to perform care correctly, various care check-in plans and care video tutorials have been designed in existing technical solutions for the users, so as to provide skin care guidance and a function of reminding manually setting for the users.

### SUMMARY

According to a first aspect, this application provides a skin care check-in method, where the skin care check-in method is applied to an electronic device and includes:
receiving a first operation that is input by a user;
in response to the first operation, displaying at least one skin care check-in plan in which the user participates;
obtaining first target information, where the first target information includes one or more of time, a location of the electronic device, a movement status of the user, and a schedule of the electronic device;
determining, based on the first target information, whether a preset implementation condition for implementing the at least one skin care check-in plan is met; and
if a determining result is that the preset implementation condition for implementing the at least one skin care check-in plan is met, displaying first reminder information, where the first reminder information is used to prompt the user to implement the at least one skin care check-in plan.

According to the skin care check-in method provided in this application, the electronic device determines a current status of the user based on the first target information; determines, based on the current status of the user, whether the user meets a condition for implementing a skin care check-in behavior; and prompts the user to perform the skin care check-in when the skin care condition is met, or delay the check-in to a time period in which the user meets the skin care condition, thereby reducing a workload of the user and improving user experience.

In a possible implementation, the skin care check-in method further includes:
receiving a second operation that is input by the user;
in response to the second operation, obtaining at least one behavior or action of the user; and
determining whether the at least one behavior or action matches a corresponding demonstration action, where the corresponding demonstration action is a demonstration action in the at least one skin care check-in plan; and
if a determining result is that the at least one behavior or action matches the corresponding demonstration action, displaying second reminder information, where the second reminder information is used to prompt that the at least one skin care check-in plan is completed; or
if a determining result is that the at least one behavior or action does not match the corresponding demonstration action, displaying third reminder information, where the third reminder information is used to prompt that the at least one skin care check-in plan is not completed.

In this embodiment, the second operation indicates an operation of turning on a camera by the user. The electronic device may obtain a behavior or an action of the user based on the operation of the user, and perform matching between the behavior or the action of the user and a demonstration behavior or action of a skin care check-in plan. When the behavior action of the user matches the demonstration behavior or action in the skin care check-in plan, the check-in is automatically completed, and the user does not need to manually perform the check-in. Therefore, user experience is improved. In a possible implementation, the skin care check-in method further includes:
if a determining result is that the preset implementation condition for implementing the at least one skin care check-in plan is not met, displaying fourth reminder information, where the fourth reminder information is used to prompt the user to determine whether to delay implementation of the at least one skin care check-in plan.

In this embodiment, if the user does not meet the skin care check-in condition, the user may be reminded to perform the skin care check-in in a time period in which skin care check-in can be implemented, thereby further improving user experience.

In a possible implementation, the skin care check-in method further includes:
receiving a third operation that is input by the user; and
in response to the third operation, re-obtaining the first target information after predetermined time, and determining, based on the re-obtained first target information, whether the preset implementation condition for implementing the at least one skin care check-in plan is met.

In this embodiment, the third operation indicates an operation of adjusting reminder time by the user, so that the user re-obtains the first target information after time predetermined by the user, and determines whether the preset implementation condition is met, to re-remind the user to perform the skin care check-in. In this solution, user experience is improved while the user check-in is monitored.

In a possible implementation, the skin care check-in method further includes:
if a determining result is that the preset implementation condition is not met, re-obtaining the first target information after predetermined time, and determining, based on the re-obtained first target information, whether the preset implementation condition for implementing the at least one skin care check-in plan is met.

In this implementation, the user does not need to adjust the time. Instead, the electronic device automatically obtains the first target information at the predetermined time, and determines whether user meets the preset implementation condition, to determine whether to remind the user to perform the skin care check-in, thereby further improving user experience. In a possible implementation, the skin care check-in method further includes:
obtaining second target information, where the second target information includes at least one of time, a location of the electronic device, and content of an application;
determining, based on the second target information, whether the user has performed a forbidden behavior in the at least one skin care check-in plan; and
if a determining result is that the forbidden behavior is implemented, displaying fifth reminder information, where the fifth reminder information is used to remind the user that the forbidden behavior is implemented or the skin care check-in plan is not completed.

In this embodiment, the electronic device may determine, based on the obtained second target information, whether the user has performed the forbidden behavior in the skin care check-in plan, and may remind the user or punish the user according to a determining result, so as to monitor the user.

In a possible implementation, the skin care check-in method further includes:
receiving a fourth operation that is input by the user;
in response to the fourth operation, obtaining a facial image of the user;
obtaining a facial image analysis result based on the facial image, and displaying at least one skin care check-in plan based on the facial image analysis result;
receiving a fifth operation that is input by the user, where the fifth operation is used to select a skin care check-in plan in which the user does not participate; and
in response to the received fifth operation, adding the skin care check-in plan.

In this embodiment, the user may participate in the skin care check-in plan as needed based on a skin test result of the electronic device, thereby improving user experience.

According to a second aspect, this application provides an electronic device, including a processor and a storage device, where the storage device stores an application, and when the application is run by the processor, the electronic device is enabled to perform the method according to the first aspect.

According to a third aspect, this application provides a computer-readable storage medium, where the computer-readable storage medium stores a computer program, and when the computer program is run on a computer, the computer is enabled to perform the method according to the first aspect.

According to a fourth aspect, this application provides a computer program, where when the computer program is executed by a computer, the computer program is used to perform the method according to the first aspect.

### BRIEF DESCRIPTION OF DRAWINGS

FIG. 1 is a schematic diagram of a structure of an electronic device 100 according to an embodiment of this application;
FIG. 2 is a block diagram of a software architecture of an electronic device 100 according to an embodiment of this application;
FIG. 3(a) to FIG. 3(c) show graphical interfaces of a skin care check-in method according to an embodiment of this application;
FIG. 4(a) to FIG. 4(e) show graphical interfaces of another skin care check-in method according to an embodiment of this application;
FIG. 5(a) to FIG. 5(l) show graphical interfaces of still another skin care check-in method according to an embodiment of this application;
FIG. 6(a) to FIG. 6(e) show graphical interfaces of another skin care check-in method according to an embodiment of this application;
FIG. 7(a) and FIG. 7(b) show graphical interfaces of still another skin care check-in method according to an embodiment of this application;
FIG. 8(a) and FIG. 8(b) show graphical interfaces of still another skin care check-in method according to an embodiment of this application;
FIG. 9(a) and FIG. 9(b) show graphical interfaces of still another skin care check-in method according to an embodiment of this application;
FIG. 10(a) and FIG. 10(b) show graphical interfaces of still another skin care check-in method according to an embodiment of this application;
FIG. 11 is a flowchart of a skin care check-in method according to an embodiment of this application;
FIG. 12 is a flowchart of a skin care check-in method according to another embodiment of this application; and
FIG. 13 is a flowchart of a skin care check-in method according to another embodiment of this application.

### DESCRIPTION OF EMBODIMENTS

The following describes technical solutions in embodiments of this application with reference to accompanying drawings in embodiments of this application. In descriptions in embodiments of this application, "/" means "or" unless otherwise specified. For example, A/B may represent A or B. In this specification, "and/or" describes only an association relationship for describing associated objects and represents that three relationships may exist. For example, A and/or B may represent the following three cases: Only A exists, both A and B exist, and only B exists.

In the following, the terms "first" and "second" are used merely for descriptive purposes, and cannot be understood as indicating or implying relative importance or implicitly indicating the quantity of indicated technical features. Therefore, a feature limited by "first" or "second" may explicitly or implicitly include one or more features. In the descriptions of embodiments of this application, unless otherwise specified, "a plurality of" means two or more than two.

A skin test activity is to detect features of facial skin of a user, such as fine lines, nasolabial folds, dark circles, red areas, pimples, pores, pigmentation, and blackheads, and evaluate, score, and grade the user's facial skin using methods such as pattern recognition, image analysis, machine learning, and deep learning, so as to provide corresponding care suggestions. The user also has an urgent demand for skin care methods in addition to skin detection.

Various care plans and care video tutorials have been designed to help the users develop facial care habits and provide guidance for the users to perform care correctly. After joining a skin care check-in plan, the user is prompted to enter reminder time, and a message is pushed to the user by the background at the specified time, to remind the user to perform skin care. After receiving the reminder information, the user performs skin care operations by following a skin care video tutorial. After completing the skin care operations, the user needs to enter the specified page to perform check-in to complete the check-in process. If the user does not perform the check-in to complete the check-in process, a system considers that the user has not completed the check-in on the current day.

In the foregoing skin care check-in solution, the reminder time is too fixed, and the user may be driving or on the way to work at the set time and does not meet a skin care condition. Consequently, an effect of reminding the user of skin care is not good. In addition, after completing skin care, the user needs to perform check-in on the guidance page, which brings a certain workload to the user. In addition, in the foregoing skin care check-in solution, a skin care behavior of the user is not tracked, and it cannot be perceived whether the user has performed an improper skin care behavior, and cannot prompt the user to terminate in a timely manner if the user has performed an improper skin care behavior.

An embodiment of this application provides a skin care check-in method. A user may select a skin care check-in plan to join; it may be determined, based on a current status of the user, whether a skin care check-in behavior condition is met; if the skin care condition is met, the user is prompted to perform skin care check-in; if the skin care condition is not met, the user is prompted to delay the skin care to a time period in which the skin care condition is met; when the user performs skin care check-in, the user's action is identified to determine whether the user's skin care behavior meets the action requirements; and if the user's skin care behavior meets the action requirements, the skin care check-in is automatically completed. According to the technical solution provided in this application, the user can be intelligently reminded of skin care based on the current status of the user, and the skin care behavior of the user can be monitored. In addition, the check-in for the skin care behavior implemented by the user can be automatically performed, so that time costs generated when the user performs the skin care behavior are reduced, and user satisfaction is improved.

The skin care check-in method provided in this embodiment of this application may be applied to electronic devices such as a mobile phone, a tablet computer, a wearable device, an in-vehicle device, an augmented reality (augmented reality, AR)/ virtual reality (virtual reality, VR) device, a notebook computer, an ultra-mobile personal computer (ultra-mobile personal computer, UMPC), a netbook, and a personal digital assistant (personal digital assistant, PDA).

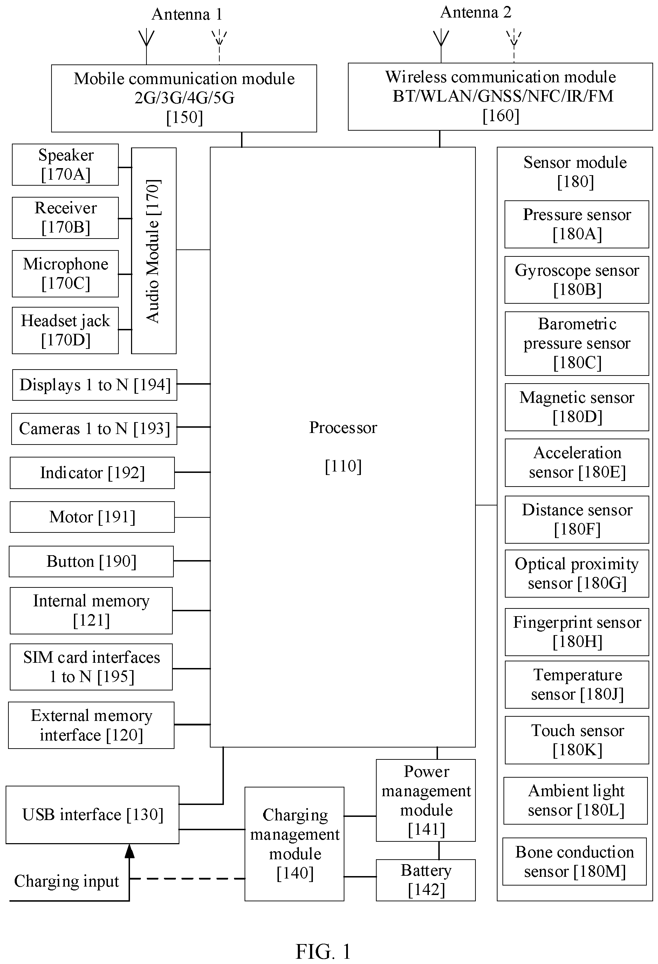

FIG. 1 is a schematic diagram of a structure of an electronic device 100 according to an embodiment of this application.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like. It may be understood that the structure shown in this embodiment of the present invention does not constitute a specific limitation on the electronic device 100. In some other embodiments of this application, the electronic device 100 may include more or fewer components than those shown in the figure, combine some components, split some components, or have different component arrangements. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors. The controller may generate an operation control signal based on an instruction operation code and a time sequence signal, to complete control of instruction reading and instruction execution.

A memory may be further disposed in the processor 110, and is configured to store instructions and data. It may be understood that the memory in the processor may store one or more instructions. When the processor executes the one or more instructions, the electronic device may determine, based on information received by another electronic device or information reported by each sensor of the electronic device 100, whether the user currently meets a preset implementation condition for implementing the skin care check-in plan described below, or determine whether a behavior or an action of the user matches a demonstration action in the skin care check-in plan, or determine whether the user has performed a forbidden behavior.

In some embodiments, the memory in the processor 110 is a cache memory. The memory may store an instruction or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

A wireless communication function of the electronic device 100 may be implemented by using the antenna 1, the antenna 2, the mobile communication module 150, the wireless communication module 160, the modem processor, the baseband processor, and the like. The electronic device 100 may communicate with and exchange information with another peripheral by using a wireless communication function. For example, the electronic device 100 communicates with a device such as a makeup mirror, a head unit, or a sports band by using a wireless communication function, to obtain information obtained by the device such as the makeup mirror or the sports band.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna in a wireless local area network.

The wireless communication module 160 may provide a wireless communication solution that is applied to the electronic device 100 and that includes a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs that execute a program instruction to generate or change display information. The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the electronic device 100 may include one or N displays 194, where N is a positive integer greater than 1.

The electronic device 100 may implement a photographing function through the camera 193, the ISP, the video codec, the GPU, the display 194, the application processor and the like.

The ISP is configured to process data fed back by the camera 193. For example, during photographing, a shutter is pressed, and light is transmitted to a photosensitive element of the camera by using a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 193.

The camera 193 is configured to capture a still image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP for converting the electrical signal into a digital image signal. The ISP outputs the digital image signal to the DSP for processing. The DSP converts the digital image signal into an image signal in a standard format such as RGB or YUV. In some embodiments, the electronic device 100 may include one or N cameras 193, where N is a positive integer greater than 1.

The external memory interface 120 may be used to connect to an external storage card, for example, a micro SD card, to extend a storage capability of the electronic device 100. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The internal memory 121 may include a program storage area and a data storage area. The program storage area may store an operating system, an application required for at least one function (such as a sound playing function and an image display function), and the like. The data storage area may store data (such as audio data and a phone book) created when the electronic device 100 is used, and the like. In addition, the internal memory 121 may include a high-speed random access memory, or may include a nonvolatile memory, for example, at least one magnetic disk storage device, a flash memory, or a universal flash storage (universal flash storage, UFS). The processor 110 performs various function applications and data processing of the electronic device 100 by running the instructions stored in the internal memory 121 and/or instructions stored in a memory provided in the processor.

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal output, and is also configured to convert an analog audio input into a digital audio signal. The audio module 170 may be further configured to encode and decode an audio signal. In some embodiments, the audio module 170 may be disposed in the processor 110, or some functional modules of the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an electrical audio signal into a sound signal. When a call is answered or speech information is received through the electronic device 100, the receiver 170B may be put close to a human ear to listen to a voice. The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, so as to implement a directional recording function and the like.

The headset jack 170D is configured to connect to a wired headset. The headset jack 170D may be the USB interface 130, or may be a 3.5 mm open mobile terminal platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch position based on a detection signal of the pressure sensor 180A. In some embodiments, touch operations that are performed in a same touch position but have different touch operation intensity may be corresponding to different operation instructions. For example, when a touch operation whose touch operation intensity is less than a first pressure threshold is performed on a messages application icon, an instruction for viewing an SMS message is performed. When a touch operation whose touch operation intensity is greater than or equal to the first pressure threshold is performed on the messages application icon, an instruction for creating a new SMS message is performed.

The gyroscope sensor 180B may be configured to determine a moving posture of the electronic device 100. In some embodiments, angular velocities of electronic device 100 around three axes (namely x, y, and z axes) may be determined by using the gyroscope sensor 180B. The gyroscope sensor 180B may be configured to implement image stabilization during photographing. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse movement, to implement image stabilization. The gyroscope sensor 180B may also be used in a navigation scenario and a somatic game scenario.

The barometric pressure sensor 180C is configured to measure barometric pressure. In some embodiments, the electronic device 100 calculates an altitude through the barometric pressure measured by the barometric pressure sensor 180C, to assist in positioning and navigation.

The magnetic sensor 180D includes a Hall sensor. The electronic device 100 may detect opening and closing of a flip cover by using the magnetic sensor 180D. In some embodiments, when the electronic device 100 is a clamshell phone, the electronic device 100 may detect opening and closing of a flip cover based on the magnetic sensor 180D. Further, a feature such as automatic unlocking of the flip cover is set based on a detected opening or closing state of the leather case or a detected opening or closing state of the flip cover.

The acceleration sensor 180E can detect magnitudes of acceleration of the electronic device 100 in various directions (generally three axes). When the electronic device 100 is still, the acceleration sensor may detect a magnitude and a direction of gravity. The acceleration sensor may be further configured to identify a posture of the electronic device, and is used in an application such as switching between a landscape mode and a portrait mode or a pedometer. The acceleration sensor may assist the electronic device 100 in obtaining at least one piece of target information in this application. For example, the acceleration sensor may calculate an amplitude and a force of a movement of the user by using an acceleration value. After obtaining the target information, the acceleration sensor may report the target information to the application processor. The application processor determines, according to the target information reported by the acceleration sensor, whether the user meets a preset implementation condition for implementing a skin care check-in plan, or whether a behavior or an action of the user matches a demonstration action in the skin care check-in plan.

The distance sensor 180F is configured to measure a distance. The electronic device 100 may measure the distance in an infrared manner or a laser manner. In some embodiments, in a photographing scenario, the electronic device 100 may measure a distance through the distance sensor 180F to implement quick focusing.

The optical proximity sensor 180G may include, for example, a light-emitting diode (LED) and an optical detector such as a photodiode. The light emitting diode may be an infrared light emitting diode. The electronic device 100 emits infrared light by using the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that the user holds the electronic device 100 close to an ear for a call, to automatically turn off a screen for power saving. The optical proximity sensor 180G may also be used in a holster mode or a pocket mode to automatically perform screen unlocking or locking.

The ambient light sensor 180L is configured to sense ambient light brightness. The electronic device 100 may adaptively adjust brightness of the display 194 based on the sensed ambient light brightness. The ambient light sensor 180L may also be configured to automatically adjust white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like. The temperature sensor 180J is configured to detect a temperature. In some embodiments, the electronic device 100 executes a temperature processing policy through the temperature detected by the temperature sensor 180J. For example, when the temperature reported by the temperature sensor 180J exceeds a threshold, the electronic device 100 reduces performance of a processor nearby the temperature sensor 180J, to reduce power consumption for thermal protection. In some other embodiments, when the temperature is less than another threshold, the electronic device 100 heats the battery 142 to prevent the electronic device 100 from being shut down abnormally due to a low temperature. In some other embodiments, when the temperature is lower than still another threshold, the electronic device 100 boosts an output voltage of the battery 142 to avoid abnormal shutdown caused by a low temperature.

The touch sensor 180K is also referred to as a "touch device". The touch sensor 180K may be disposed on the display 194, and the touch sensor 180K and the display 194 form a touchscreen, which is also referred to as a "touchscreen". The touch sensor 180K is configured to detect a touch operation performed on or near the touch sensor. The touch sensor may transfer the detected touch operation to the application processor to determine a type of the touch event. A visual output related to the touch operation may be provided through the display 194. In some other embodiments, the touch sensor 180K may also be disposed on a surface of the electronic device 100 in a position different from that of the display 194. The touch sensor may assist the electronic device 100 in obtaining at least one piece of target information in this application. For example, the touch sensor may detect a touch action of the user to determine whether the user is currently using the electronic device 100, and report the touch information of the user to the application processor. The application processor determines, based on the target information reported by the touch sensor, whether the user meets a preset implementation condition for implementing a skin care check-in plan, or whether the behavior or the action of the user matches a demonstration action in the skin care check-in plan, or whether the user's behavior is related to a forbidden behavior of the skin care check-in plan.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a body pulse to receive a blood pressure beating signal. In some embodiments, the bone conduction sensor 180M may also be disposed in the headset, to obtain a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function. The bone conduction sensor 180M may assist the electronic device 100 in obtaining at least one piece of target information in this application. For example, the bone conduction sensor 180M may detect heart rate information of a user, to determine whether the user is currently doing exercise and a movement intensity, and report a target to the application processor. The application processor determines, based on the target information reported by the bone conduction sensor 180M, whether the user meets a preset implementation condition for implementing a skin care check-in plan, or whether a behavior or an action of the user matches a demonstration action in the skin care check-in plan, or whether the user's behavior is related to the forbidden behavior of the skin care check-in plan.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt or a touch vibration feedback. For example, touch operations performed on different applications (such as photographing and audio playing) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may be further customized.

The indicator 192 may be an indicator light, and may be configured to indicate a charging status and a power change, or may be configured to indicate a message, a missed call, a notification, and the like.

A software system of the electronic device 100 may use a layered architecture, an event-driven architecture, a microkernel architecture, a micro service architecture, or a cloud architecture. In embodiments of the present invention, an Android system of a layered architecture is used as an example to illustrate a software architecture of the electronic device 100.

It may be understood that the structure shown in FIG. 1 does not constitute any specific limitation on the electronic device 100. In some other embodiments of the present invention, the electronic device 100 may include more or fewer components than those shown in the figure, or some components may be combined, or some components may be split, or different component arrangements may be used. The components shown in the figure may be implemented by hardware, software, or a combination of software and hardware.

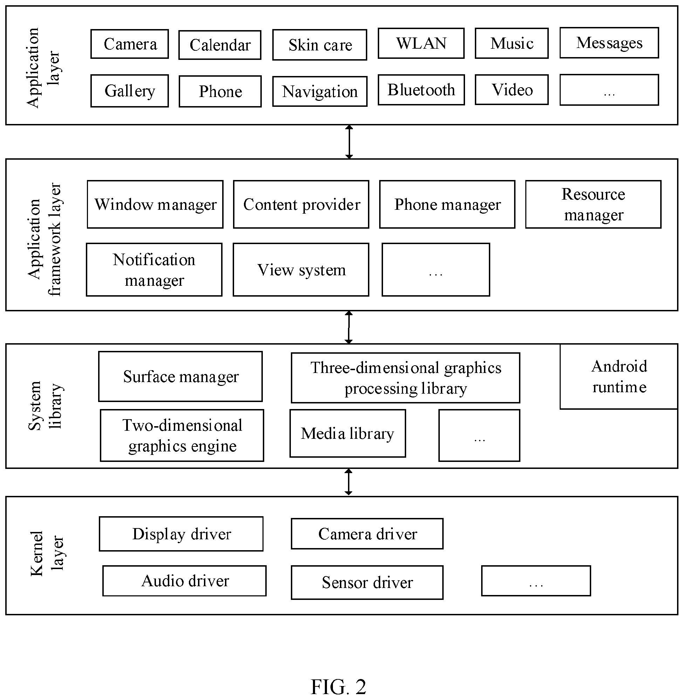

FIG. 2 is a block diagram of a software architecture of an electronic device 100 according to an embodiment of the present invention.

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom. The application layer may include a series of application packages.

As shown in FIG. 2, the application packages may include applications such as Camera, Skin care, Gallery, Calendar, Phone, Map, Navigation, WLAN, Bluetooth, Music, Video, and Messages. The application framework layer provides an application programming interface (application programming interface, API) and a programming framework for an application at the application layer. The application framework layer includes some predefined functions.

As shown in FIG. 2, the application framework layer may include a window manager, a content provider, a view system, a phone manager, a resource manager, a notification manager, and the like.

The window manager is configured to manage a window program. The window manager may obtain a size of a display, determine whether there is a status bar, perform screen locking, take a screenshot, and the like.

The content provider is configured to store and obtain data, and enable the data to be accessed by an application. The data may include a video, an image, audio, calls that are made and answered, a browsing history and bookmarks, an address book, and the like.

The view system includes visual controls such as a control for displaying a text and a control for displaying a picture. The view system may be used to construct an application. A display interface may include one or more views. For example, a display interface including an SMS message notification icon may include a text display view and a picture display view.

The phone manager is configured to provide a communication function for the electronic device 100, for example, management of a call status (including answering, declining, or the like).

The resource manager provides various resources such as a localized character string, an icon, a picture, a layout file, and a video file for an application.

The notification manager enables an application to display notification information in a status bar, and may be configured to convey a notification message. The notification manager may automatically disappear after a short pause without requiring a user interaction. For example, the notification manager is configured to notify download completion, provide a message notification, and the like. The notification manager may alternatively be a notification that appears in a top status bar of the system in a form of a graph or a scroll bar text, for example, a notification of an application that is run on a background, or may be a notification that appears on the screen in a form of a dialog window. For example, text information is prompted in the status bar, an alert sound is played, the electronic device vibrates, or the indicator light blinks.

The Android runtime includes a kernel library and a virtual machine. The Android runtime is responsible for scheduling and management of the Android system.

The kernel library includes two parts: a function that needs to be called in Java language and a kernel library of Android.

The application layer and the application framework layer run on the virtual machine. The virtual machine executes Java files of the application layer and the application framework layer as binary files. The virtual machine is configured to implement functions such as object lifecycle management, stack management, thread management, security and abnormality management, and garbage collection.

The system library may include a plurality of functional modules, such as a surface manager (surface manager), media libraries (Media Libraries), a 3D graphics processing library (for example, an OpenGL ES), and a 2D graphics engine (for example, an SGL).

The surface manager is configured to manage a display subsystem and provide fusion of 2D and 3D layers for a plurality of applications.

The media library supports playback and recording in a plurality of commonly used audio and video formats, static image files, and the like. The media libraries may support a variety of audio and video encoding formats, such as MPEG4, H.264, MP3, AAC, AMR, JPG, PNG, and the like. The three-dimensional graphics processing library is configured to implement three-dimensional graphics drawing, image rendering, compositing, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes an example of a working process of software and hardware of the electronic device 100 with reference to a scenario in which photographing is performed for skin test.

When the touch sensor 180K receives a touch operation, a corresponding hardware interrupt is sent to the kernel layer. The kernel layer processes the touch operation into an original input event (including information such as touch coordinates and a time stamp of the touch operation). The original input event is stored at the kernel layer. The application framework layer obtains the original input event from the kernel layer, and identifies a control corresponding to the input event.

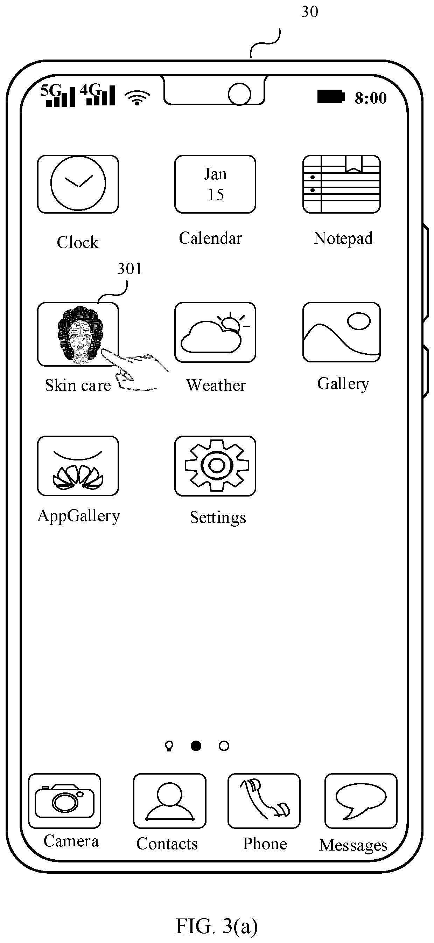

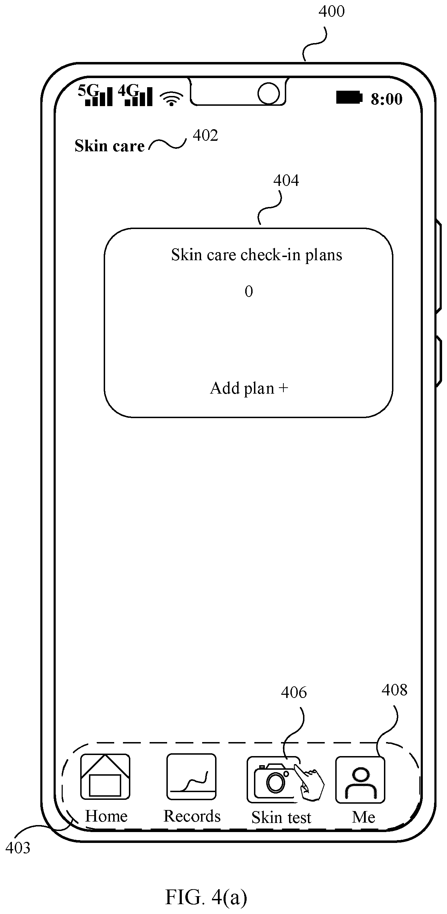

For example, the touch operation is a click operation, and a control corresponding to the click operation is a control of an application icon "skin care application 301" in the following embodiment (refer to FIG. 3a). The skin care application invokes an interface of the application framework layer to start the skin care application. If the user clicks the "Skin test" option 4031 (refer to FIG. 4a), the skin care application invokes an interface of the application framework layer, and then starts a camera driver by invoking the kernel layer, so as to capture a static image or a video by using the camera 193.

For ease of understanding, the following describes the skin care check-in method disclosed in this application with reference to a mobile phone having the structures shown in FIG. 1 and FIG. 2 as an example.

FIG. 3(a) to FIG. 3(c) show schematic diagrams of graphical interfaces (graphical user interface, GUI) of a skin care check-in method according to an embodiment of this application. FIG. 3(a) shows interface content 30 output by the mobile phone after being powered on. The interface content 30 displays a plurality of applications (application, App), such as Clock, Calendar, Settings, Notepad, Weather, AppGallery, Gallery, and a skin care application 301. It should be understood that the interface content 30 may further include more applications. This is not limited in this embodiment of this application.

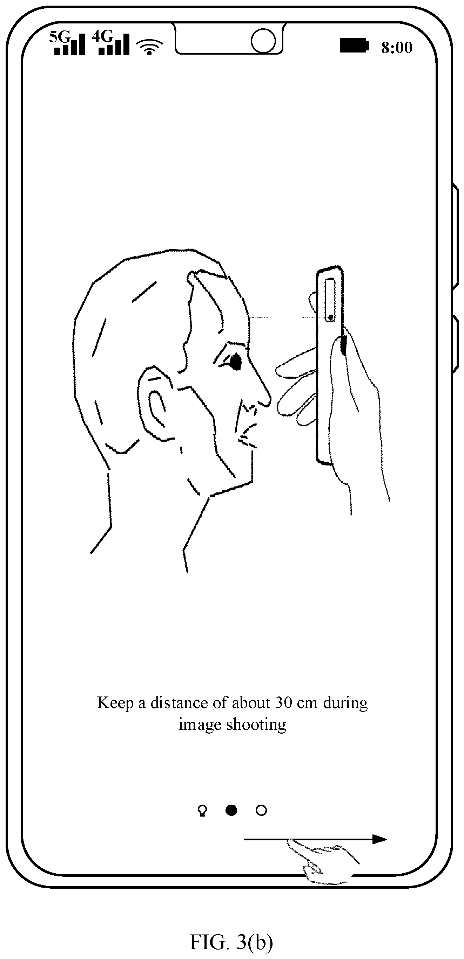

As shown in FIG. 3(a), the user clicks an icon of the skin care application 301, and the mobile phone starts the skin care application 301 in response to the click operation of the user. After the skin care application 301 is installed, when the skin care application 301 is started for the first time, some precautions for using the skin care application 301 may be displayed. FIG. 3(b) shows the precautions for using the skin care application. The precautions for using the skin care application may be explained to the user by using an image and text. As shown in FIG. 3(b), a distance of about 30 cm should be kept when a built-in camera of the mobile phone is used to take a photo for skin test.

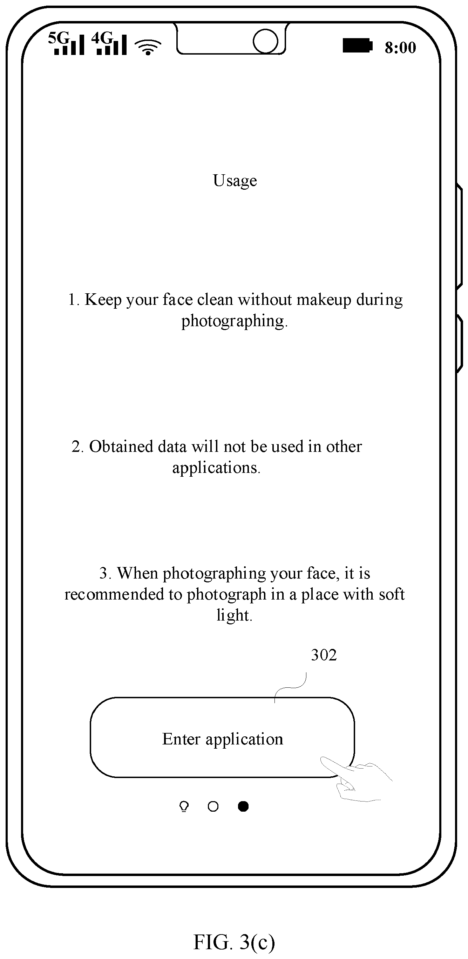

The user can slide in a specified direction on the screen of the mobile phone to view more usage instructions of the skin care application 301. As shown in FIG. 3(c), it is recommended that a face should be clean without makeup during image shooting, that skin detection data should be not used for another application, or the like. It may be understood that, to further reduce the workload of the user, the foregoing precautions may be automatically switched.

In a possible implementation, after the skin care application 301 is started, if it is not the first time that the user enters the skin care application 301, the user may view the skin care precautions in another manner, for example, view the skin care precautions by clicking another button on the interface.

After reading the precautions, the user may click to enter the application 302, to display a home screen of the skin care application 301. FIG. 4(a) to FIG. 4(e) show graphical interfaces of still another skin care check-in method according to an embodiment of this application. FIG. 4(a) shows a home screen 400 of the skin care application. The home screen 400 of the skin care application may include a function name 402, a check-in list 404, and a navigation bar 403. The function name 402 may be used to indicate a currently started application of the local device, for example, a skin care application of the local device. The check-in list 404 indicates a check-in plan that the user has joined. The navigation bar 403 may include various function menus. As shown in FIG. 4(a), the navigation bar 403 include a "Home" button for viewing a home screen of the skin care application, a "Records" button for viewing skin care records of a user, a "Skin test" button 406 for obtaining an image and detect the image, and a "Me" button 408 for managing a personal account.

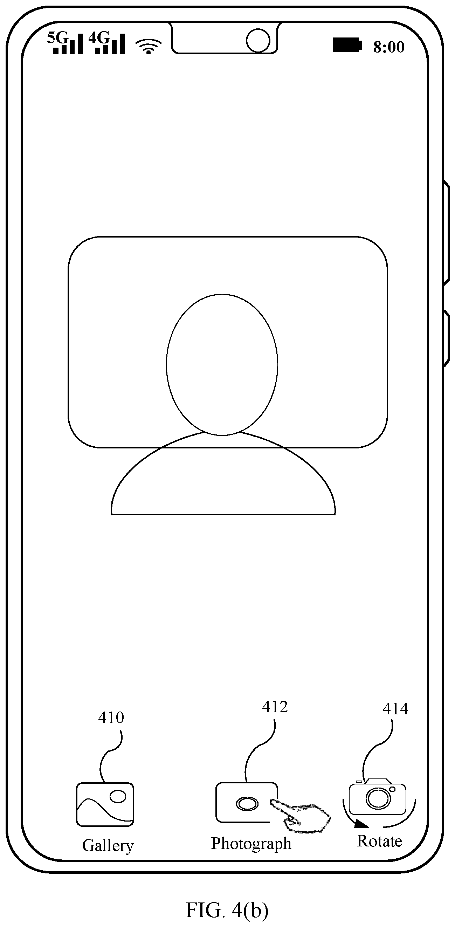

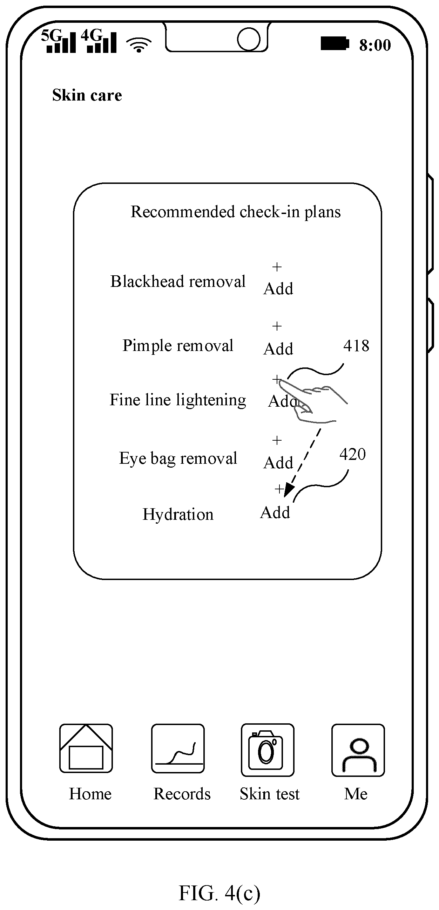

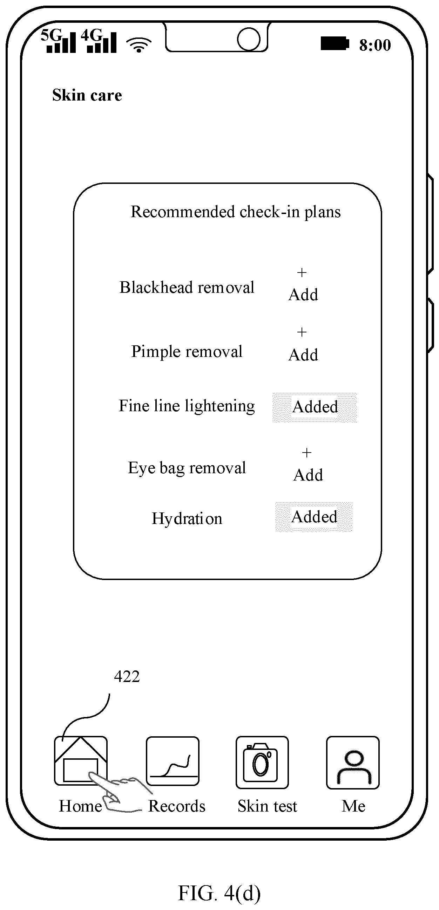

For example, the user may click the "Skin test" button 406 to display an interface for obtaining a user image. FIG. 4(b) shows an interface for obtaining a user image. A user may click a "Photograph" button 412 on the interface, so that the mobile phone shoots an image of the user in response to the click operation of the user, analyzes the shot image, and recommends a skin care check-in plan to the user based on an analysis result. If the image displayed on the current interface is an image obtained by a rear-facing camera, and the user needs to take a selfie to obtain a facial image, the user may click a "Switch" button 414 to switch from the rear-facing camera to a front-facing camera for photographing. Alternatively, the user may click a "Gallery" button 410, so that the mobile phone obtains, from a local gallery 410, an image selected by the user, and analyzes the image, so as to recommend a skin care check-in plan to the user based on an analysis result. After the skin care application analyzes the image (for a specific analysis method, refer to the following description), an interface displaying the recommended skin care check-in plan is displayed based on the analysis result. For example, if it is determined, through analysis, that a face of the user has blackheads, a blackhead check-in plan may be recommended on the displayed interface. FIG. 4(c) shows an example of recommended check-in plans such as "Blackhead removal", "Pimple removal", "Fine line lightening", "Eye bag removal", and "Hydration" to the user, and the user may select to join a check-in plan as required. In FIG. 4(c), the user clicks an "Add" button 418 to add the "Fine line lightening" check-in plan, and then clicks an "Add" button 420 to add the "Hydration" check-in plan as required. As shown in FIG. 4(d), the statuses of "Fine line lightening" and "Hydration" in the list of recommended check-in plans list changes to "Added".

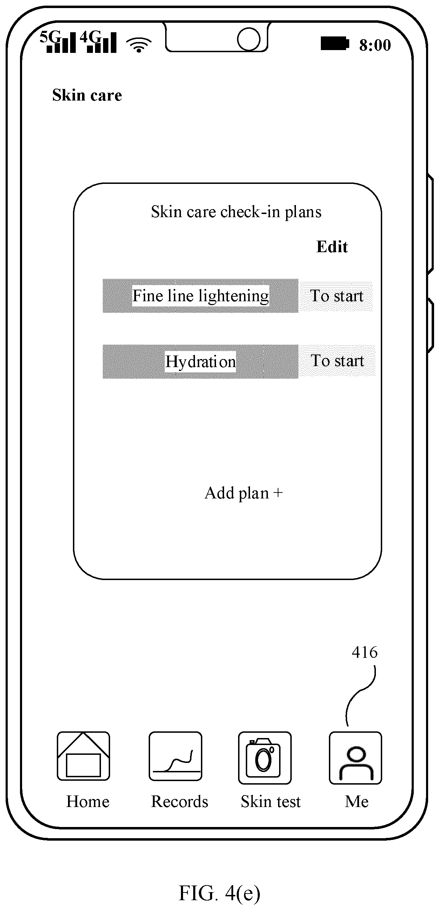

Still refer to FIG. 4(c). The user may click the "Home" button 422 to return to the home screen of the skin care application 301 and view a check-in plan that has been added to the check-in list 404. FIG. 4(e) shows the home screen of the skin care application 301 after the user adds the skin care check-in plans. Because of the foregoing adding operations of the user, two check-in plans, namely "Fine line lightening" and "Hydration", are added to the check-in list.

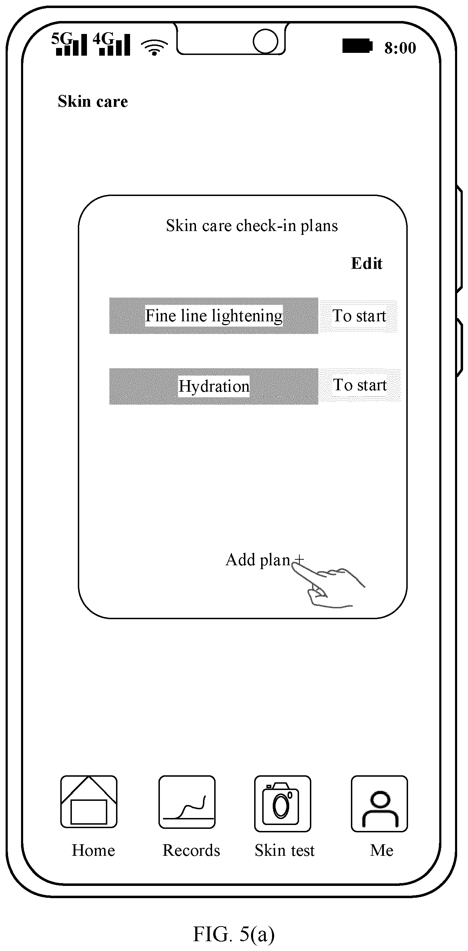

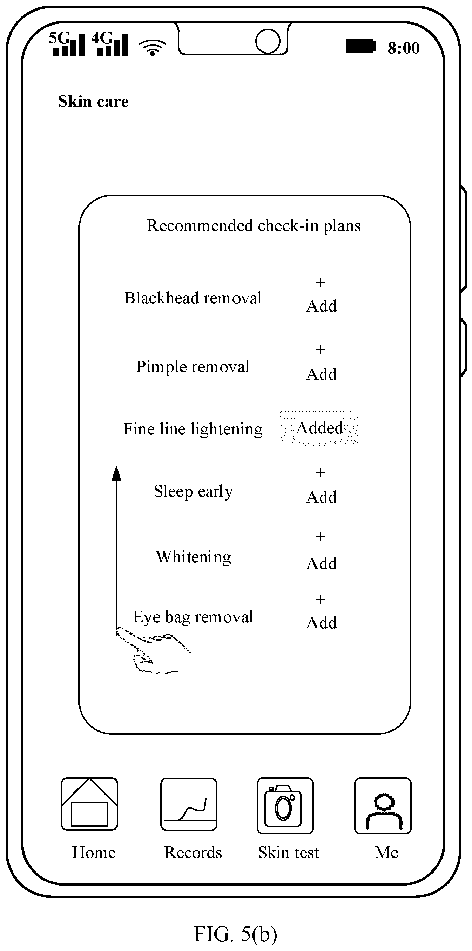

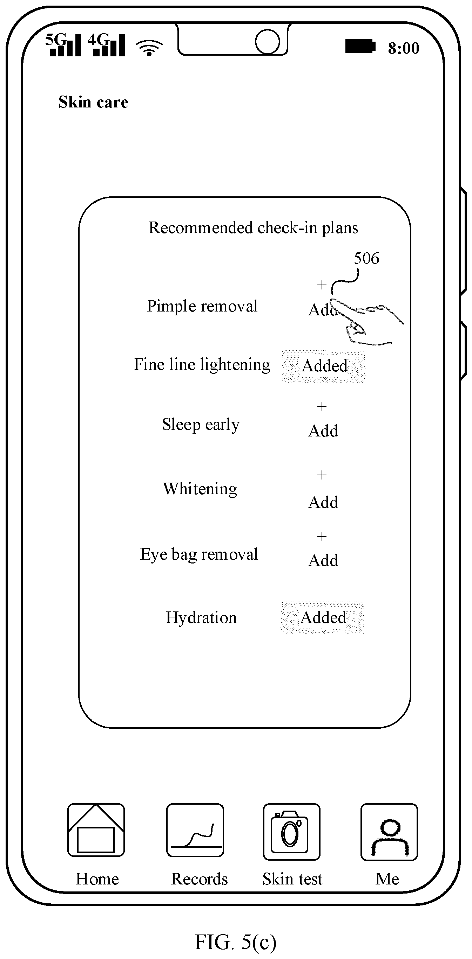

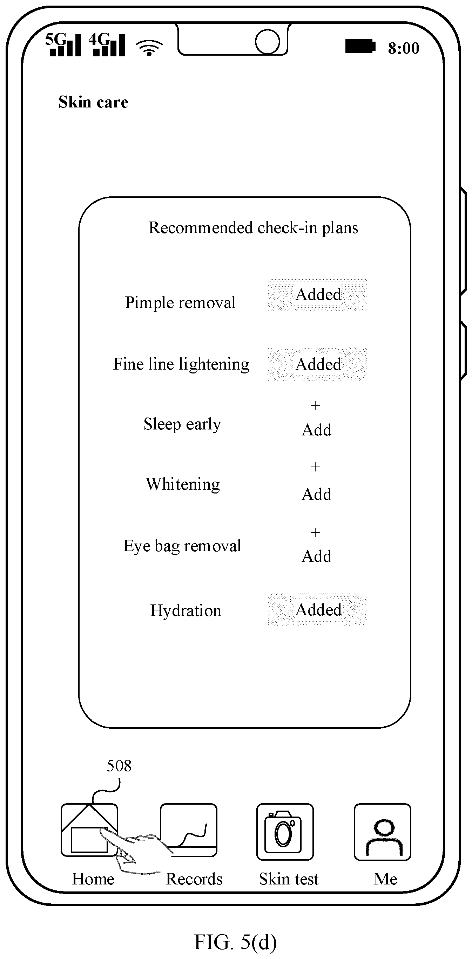

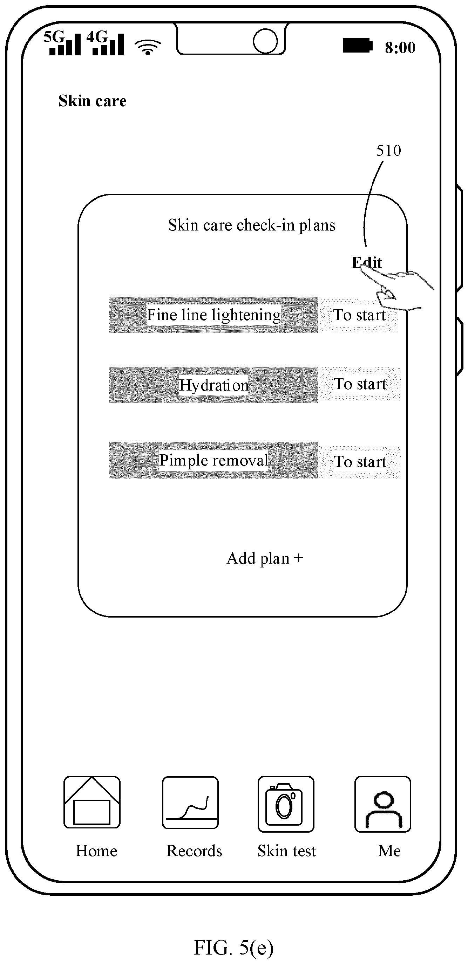

It should be noted that adding of a skin care check-in plan by the skin care application is not limited to the following manner: skin care check-in plans are recommenced to the user after a skin test, and the user selects a plan recommended by the skin care application. A skin care check-in plan may also be added in another manner. FIG. 5(a) to FIG. 5(l) show graphical interfaces of still another skin care check-in method according to an embodiment of this application. In FIG. 5(a), the user may click an "Add plan" button 504 to add a skin care check-in plan. For example, refer to FIG. 5(b). After the user clicks the "Add plan" button 504, the local device displays, in response to the click operation of the user, a graphical interface displaying all skin care check-in plans included in the skin care application. The user may select a plan as required. In FIG. 5(b), the user may slide down to view and add more skin care check-in plans. Two check-in plans, namely "Fine line lightening" and "Hydration", have been added. The user may click the "Add" button to add check-in plans other than "Fine line lightening" and "Hydration" as required. As shown in FIG. 5(c) and FIG. 5(d), the user clicks the "Add" button 506 corresponding to the "Pimple removal" check-in plan, so that the status of the "Pimple removal" check-in plan changes to "Added". After the user adds a skin care check-in plan, the check-in list on the home screen 400 of the skin care application correspondingly changes. On a graphical interface shown in FIG. 5(d), the user may click the "Home" button to view the check-in plan list on the home screen. For example, after the user clicks the "Home" button 508, a graphical interface in FIG. 5(e) is displayed, and the check-in plan list 404 on the home screen 40 displays the skin care check-in plans "Pimple removal", "Fine line lightening", and "Hydration" that have been added by the user.

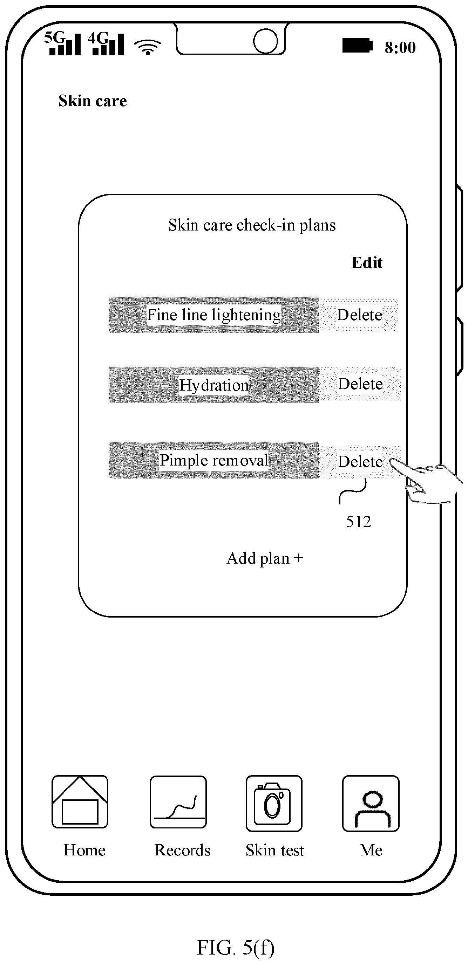

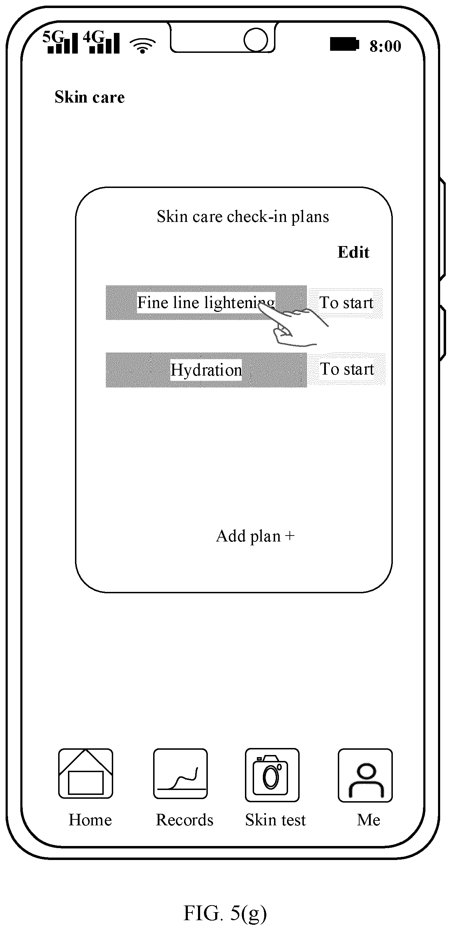

It should be noted that the user may select to delete a skin care check-in plan that has been added. As shown in FIG. 5(e), the user clicks an "Edit" button 510 to invoke an interface for deleting a skin care check-in plan, to delete an unwanted skin care check-in plan. FIG. 5(f) shows an example of an interface for deleting a check-in plan. The user clicks a "Delete" button 512 corresponding to the "Pimple removal" skin care check-in plan, to delete the "Pimple removal" skin care check-in plan that has been added. After the "Pimple removal" check-in plan is deleted, an interface shown in FIG. 5(g) is displayed.

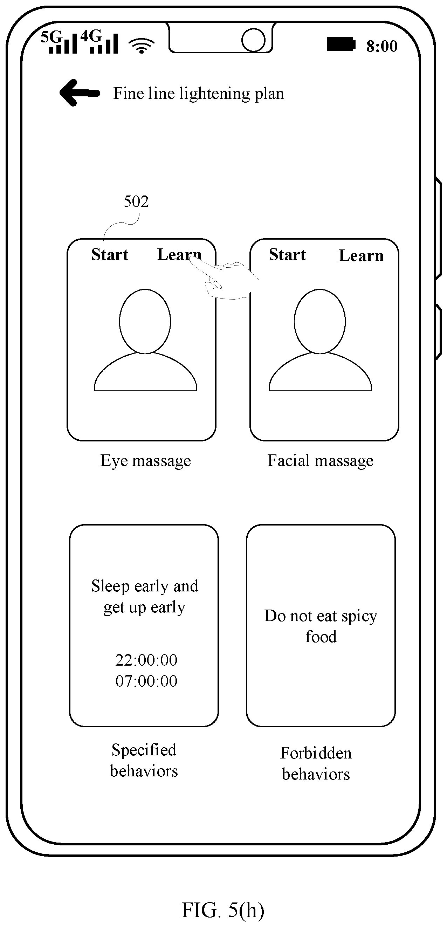

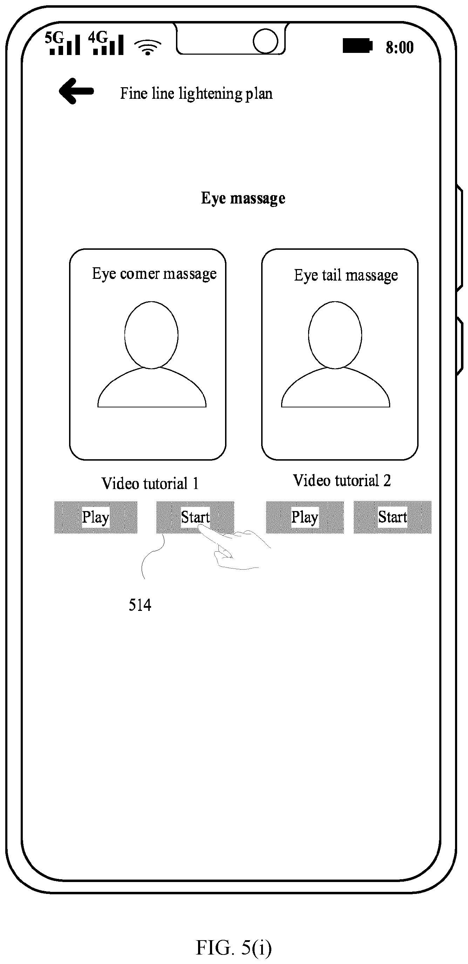

After adding a skin care check-in plan, the user can view the check-in plan in the check-in list in real time. On the graphical interface shown in FIG. 5(g), the user clicks the "Fine line lightening" button, and the skin care application displays a demonstration action in a fine line lightening check-in plan in response to the click operation of the user. It should be noted that each skin care check-in plan may be considered as a skin care mode. For example, the fine line lightening check-in plan is considered as a skin care mode, and the blackhead removal check-in plan is considered as a skin care mode. Each skin care mode includes at least one demonstration action, and the demonstration action is used to resolve or alleviate a skin problem of the user, or is used to care the skin of the user. Each demonstration action includes but is not limited to a combination of a set of continuous actions, or a behavior. For example, refer to interface content shown in FIG. 5(h). A demonstration action in the fine line lightening plan includes "Eye massage", "Facial massage", and "Sleep early and get up early". It may be understood that "Eye massage" and "Facial massage" are a combination of a set of continuous actions, and "Sleep early and get up early" is a behavior. The user may learn a combination of a set of continuous actions by watching a video. For example, the user may click a learning button in "Eye massage" to display an interface shown in FIG. 5(i), where

FIG. 5(i) includes a video tutorial on an eye massage. The user may click a play button corresponding to an eye corner massage video to watch and learn a teaching video of eye corner massage, or click a play button corresponding to an eye tail massage video to watch and learn a teaching video corresponding to an eye tail massage.

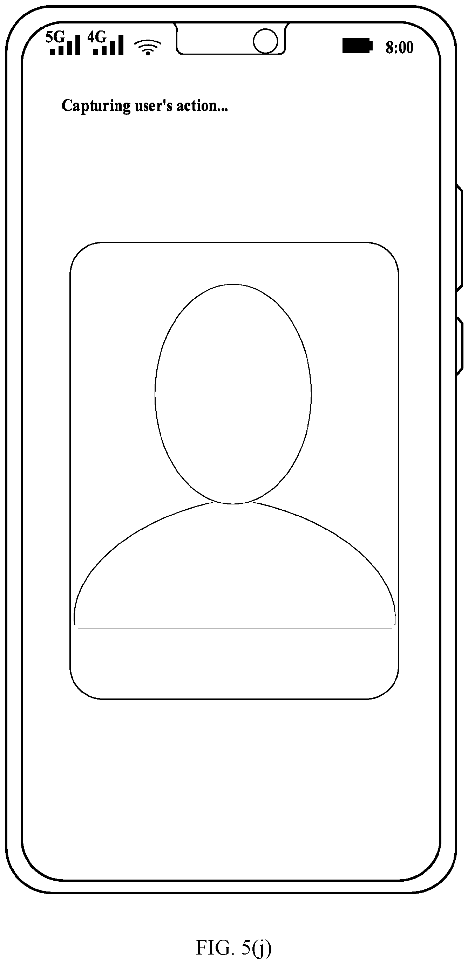

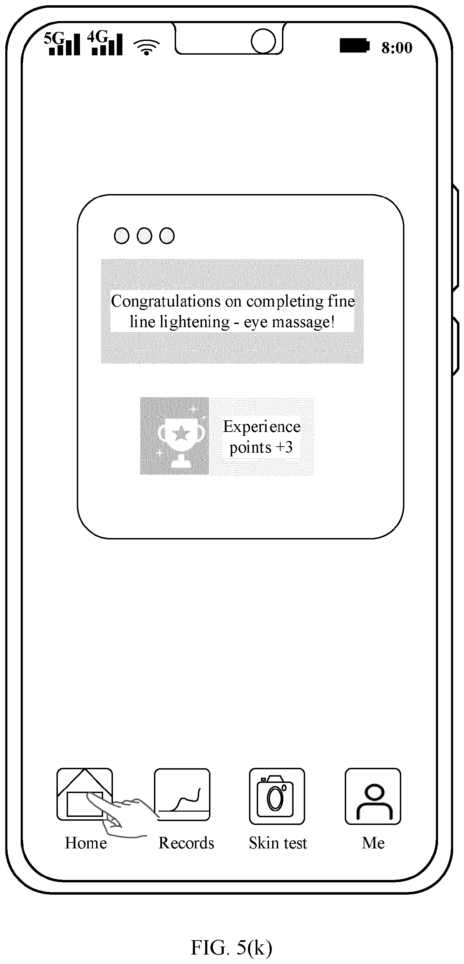

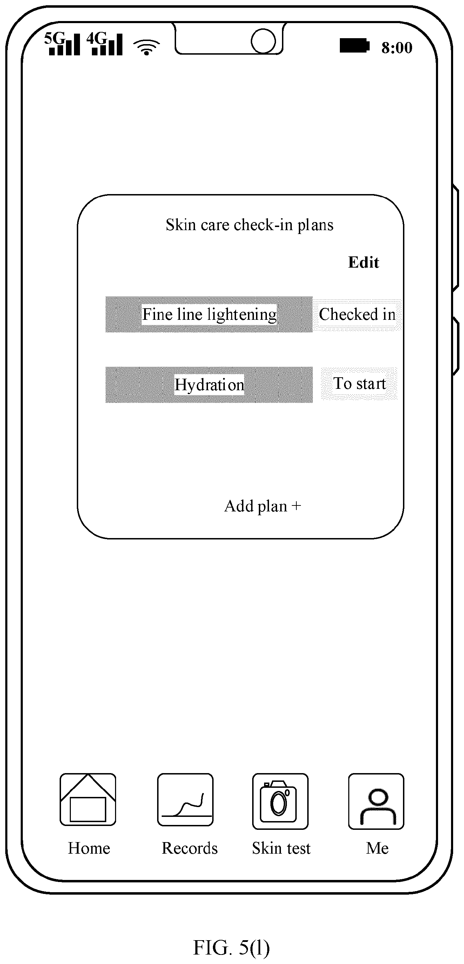

After learning the demonstration action of the skin care mode, the user may click a "Start" button 514 in FIG. 5(i), to implement the demonstration action related to the eye corner massage in the fine line lightening plan. Certainly, the user may further return to the interface shown in FIG. 5(h), and click the "Start" button 502, to implement a demonstration action related to the eye massage in the fine line lightening plan. Specifically, after the user clicks the "Start" button 514/502, a graphical interface shown in FIG. 5(j) is displayed. The skin care application invokes a camera of the mobile phone to detect and obtain a behavior and an action of the user in real time, and then performs matching between the behavior and the action of the user and the demonstration action related to an eye massage in the fine line lightening plan. If the behavior or the action performed by the user matches a demonstration action of the eye massage, the demonstration action of the eye massage is completed. In the skin care application, if the behavior or the action of the user matches at least one demonstration action, after the eye massage is completed, the status of the fine line lightening plan in the skin care check-in plans on the home screen of the skin care application is changed to "Checked in". For example, after the user completes the demonstration action of the eye massage in the fine line lightening plan, as shown in FIG. 5(k), the graphical interface of the skin care application reminds the user that the eye massage action in the fine line lightening plan is completed, and the user clicks the "Home" button to return to the home screen of the skin care application. For example, as shown in FIG. 5(l), the status of the fine line lightening plan correspondingly changes to "Checked in". According to the skin care check-in method provided in this embodiment, the user does not need to go to the home screen to manually change the status to "Checked in", but automatically performs matching between a behavior or an action of the user, and helps the user complete check-in when the behavior or the action implemented by the user matches the demonstration action of the eye massage, thereby improving user experience. It may be understood that, because the "Fine line lightening" plan further includes a "Facial massage" action, it may be set that the user further needs to complete the facial massage after completing the eye massage, so that the skin care application correspondingly changes the status of the "Fine line lightening" plan to "Checked in". Although the user has joined the skin care check-in plan, the user cannot perform skin care check-in at any time and at any place every day. For example, it is not suitable for the user to perform a skin care behavior when driving. In a related technology, a manner of reminding a user to perform skin care through timing is not scientific or convenient. Therefore, a more intelligent skin care application is needed.

To further reduce the check-in workload of the user, the skin care application in this embodiment of this application may provide an intelligent check-in. The intelligent check-in is performed based on a preset implementation condition. The preset implementation condition is preset by the skin care application and is used to indicate that the user currently has a condition for implementing a skin care check-in plan. Each skin care check-in plan added by the user has a specific preset implementation condition. The specific preset implementation condition includes an implementation condition required for each demonstration action in the skin care check-in plan. The preset implementation condition is determined based on first target information. The first target information includes but is not limited to related information such as a location of the user, current time, and content (such as a calendar schedule) recorded in another application. For example, the user sets, in the skin care application, that the user is reminded to perform skin care at specified time. At the specified time, the skin care application obtains location information of the user. For example, the skin care application learns that the user is currently in an office, and therefore infers that the user is likely to be working and it is not suitable for the user to perform the skin care check-in. In this case, the skin care application may remind the user whether to delay the skin care. In addition, after intelligent check-in is enabled, the skin care application can enable the camera in the background or use a camera of another electronic device connected to the local device to obtain the action or behavior images or other information of the user, so as to detect whether the user has implemented the demonstration actions in the skin care check-in plan. When detecting that the user has completed the demonstration actions in the skin care check-in plan, the skin care application automatically changes the status of the completed skin care check-in plan to "Checked in" on the home screen.

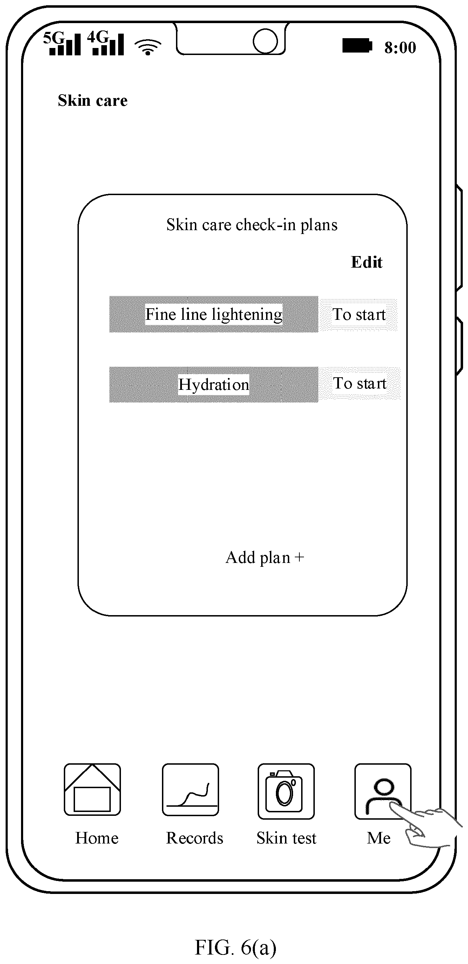

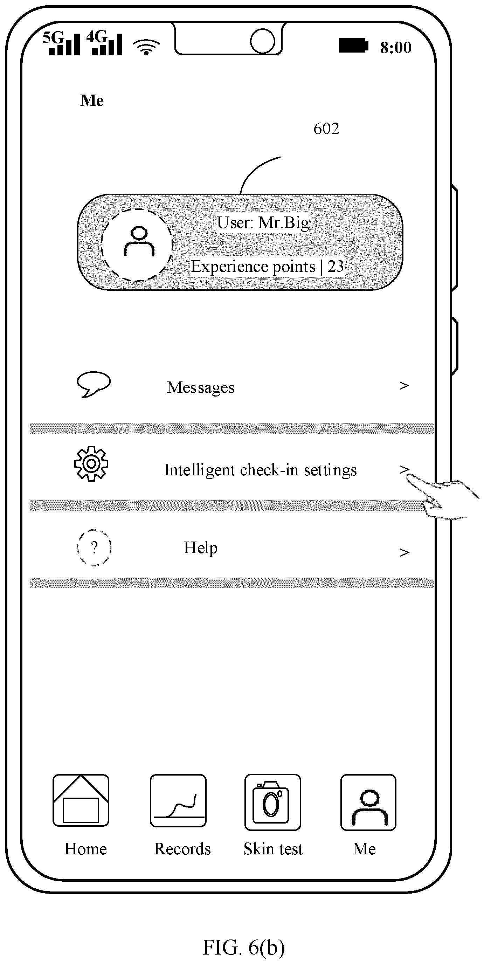

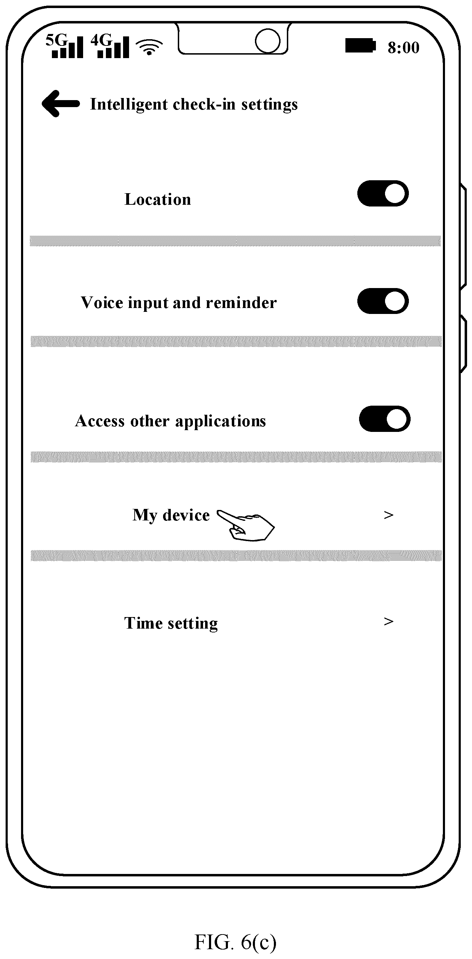

FIG. 6(a) to FIG. 6(e) are schematic diagrams of graphical interfaces of a skin care check-in method according to an embodiment of this application. A user may set intelligent check-in in the skin care application. For example, the user may click the "Me" button in the graphical interface shown in FIG. 6(a), to display the graphical interface shown in FIG. 6(b). FIG. 6(b) includes an indication bar 602 used to indicate a user account and experience points. Each time the user completes check-in for a skin care plan, the experience points may be increased. FIG. 6(b) further includes an "Intelligent check-in settings" button for setting intelligent check-in. The intelligent check-in function in the skin care application requires a permission to obtain the location of the local device and a permission to access another application. The user can click the "Intelligent check-in settings" button to display a graphical interface shown in FIG. 6(c). FIG. 6(c) includes a "Location" button for obtaining a location permission, a "Voice input and reminder" button for obtaining a voice input and a reminder permission, an "Access other applications" button for obtaining a permission for accessing other applications, a "My device" button for managing an external device connected to the local device, and a "Time setting" button for setting a time reminder for each skin care check-in plan. The intelligent check-in can be implemented only after the skin care application obtains the preceding permissions.

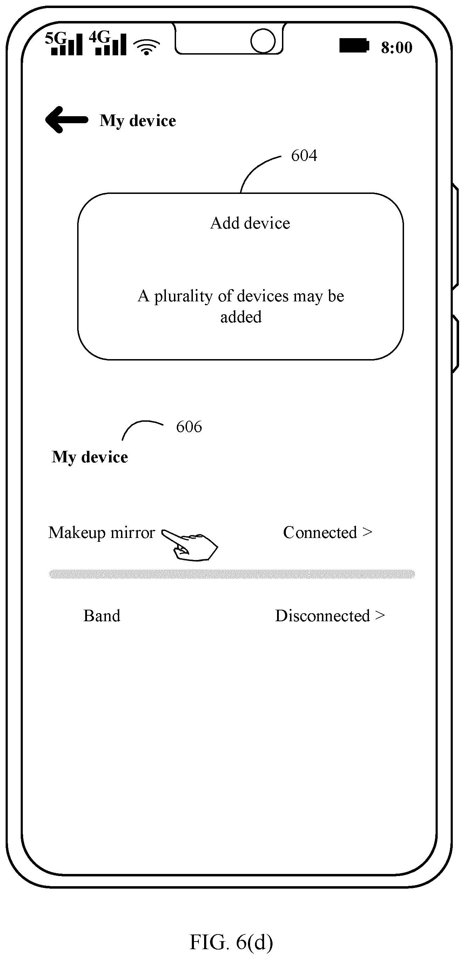

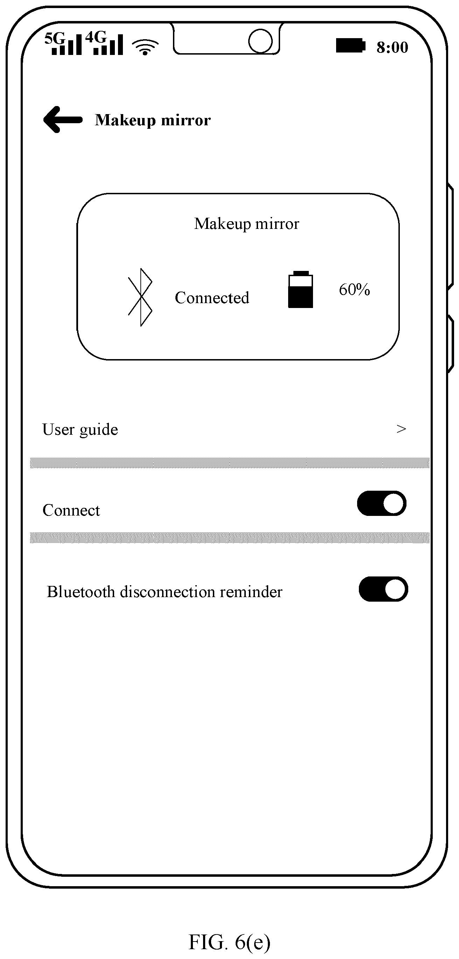

To better implement an effect of intelligent check-in, the local device works with another external device to provide the user with a more intelligent experience. Refer to FIG. 6(c). The user may click the "My device" button to manage an external device connected to the local device. The local device jumps to a graphical interface shown in FIG. 6(d) in response to the click operation of the user. The interface includes a plurality of display areas, such as an "Add device" area 604 and a "My device" area 606. The "Add device" area 604 is used to add more connectable devices, such as a sensor device. The "My device" area 606 is used to display an added device and a connection status between the added device and the local device. For example, the devices that have been added to the local device include a makeup mirror and a band, where the band is disconnected from the mobile phone. When the band is connected to the mobile phone, the band may report obtained user-related data to a skin care application in the mobile phone. The data includes but is not limited to pulse rate data, heart rate data, and the like. The user may also click the makeup mirror to view the status information and user guide of the makeup mirror. For example, as shown in FIG. 6(e), the makeup mirror is connected to the mobile phone, and the makeup mirror and the mobile phone may be connected through a Wi-Fi connection, a Bluetooth connection, or the like. The user can read a user guide to learn how to use the makeup mirror, and can enable a Bluetooth disconnection reminder. These peripheral devices connected to the local device may assist the skin care application in the local device to detect and obtain a behavior or an action and status information of the user. For example, when the user performs a skin care behavior or action, image information of the user may be obtained by using a camera in the makeup mirror, and then sent to the local device for analysis and matching. With these external devices, the user can obtain information such as an image of the user without using a camera of the local device.

It should be noted that more devices may be added to detect or obtain information required by the skin care application. This is not limited herein.

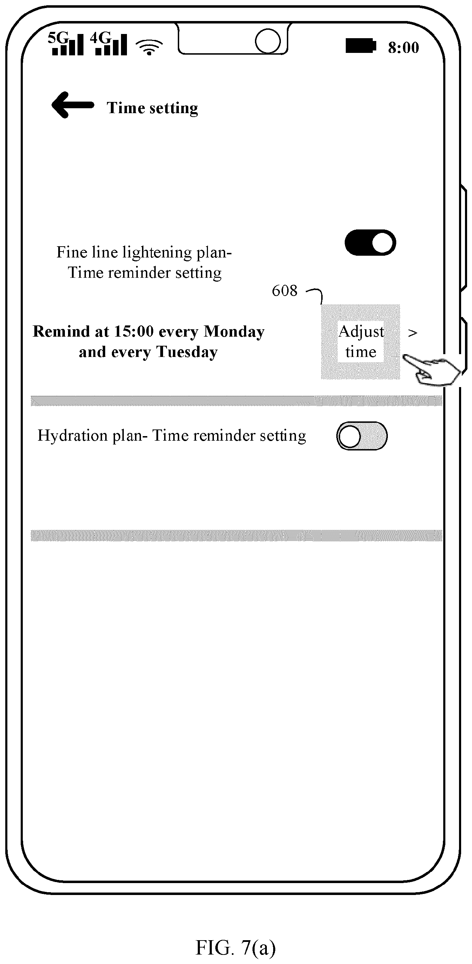

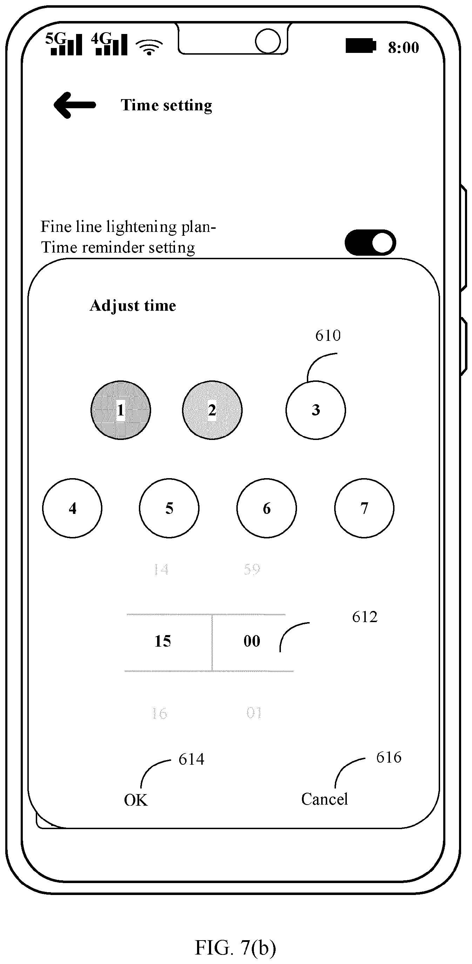

Still refer to FIG. 6(c), the user may click the "Time setting" button to set a reminder time, and the local device displays an interface shown in FIG. 7(a) in response to the click operation of the user. On the graphical interface, the user may set a time reminder for an added skin care check-in plan. For example, a time reminder has been enabled for the fine line lightening check-in plan, and it is set that the user is reminded to perform fine line lightening at 15:00 on every Monday and every Tuesday. The user may click an "Adjust time" button 608 to display an interface shown in FIG. 7(b) to adjust the time. In FIG. 7(b), the user may click a "Week" button 610 to increase or decrease a quantity of days of the fine line lightening check-in plan, or click a "Time" button 612 to adjust a daily reminder time. The user click may click an "OK" button 614 or a "Cancel" button 616 to determine whether to adjust the daily reminder time.

After setting a permission required for intelligent check-in on the interface in FIG. 6(c), the skin care application may obtain first target information at any time based on the permission, to determine whether the demonstration action in each skin care check-in plan meets a specific required preset implementation condition. For example, the skin care application obtains location information and time information of the local device, to determine whether the user meets a specific preset implementation condition for implementing the fine line lightening check-in plan. For example, when detecting that the location of the local device is the user's home and the time is a reminder time set by the user, the local device may remind the user whether to implement the fine line lightening check-in plan in the skin care application. For another example, the skin care application may read information about another device connected to the local device, to determine a status of the user. For example, the user may read information such as a heart rate and a pulse rate of the user by using a connected band or a connectable device, to determine the status of the user. For example, if the heart rate of the user is too high and the pulse beats violently, the user is likely to be doing exercising at this time, and it is not suitable for the user to perform some demonstration actions in the skin care check-in plan of the skin care application. For another example, reminder time set by the user for the fine line lightening skin care check-in is 15:00. If the location information obtained by the skin care application is a workplace of the user, or the skin care application detects, by accessing a calendar application of the user, that the user is scheduled to hold a meeting at 15:00, the user may not meet a condition for implementing "eye massage" or "Facial massage" demonstration actions in the fine line lightening check-in plan. In this case, the skin care application does not remind the user to perform a fine line lightening check-in plan in the skin care application, or reminds the user whether to perform skin care at a next time node.

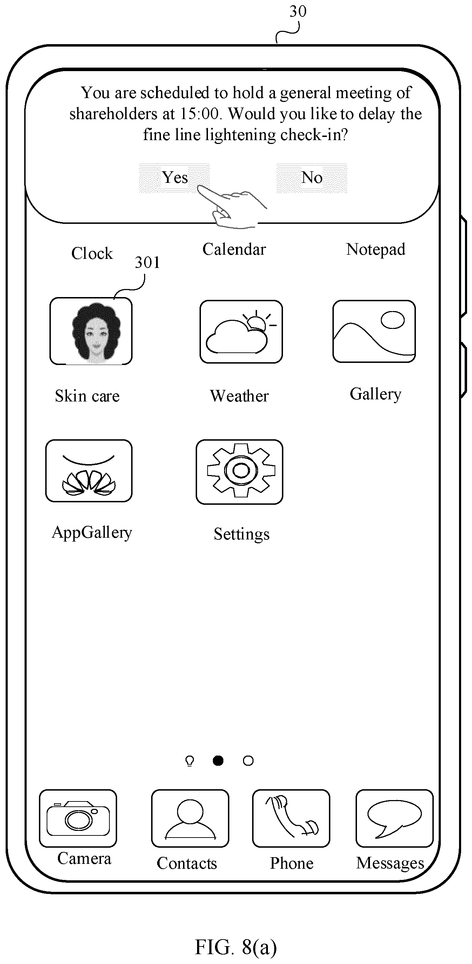

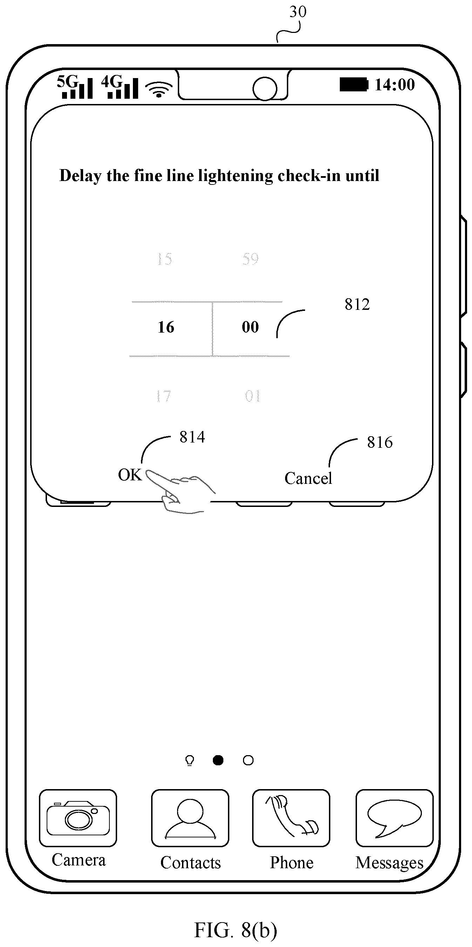

FIG. 8(a) and FIG. 8(b) show graphical interfaces of still another skin care check-in method according to an embodiment of this application. Specifically, FIG. 8(a) and FIG. 8(b) show example diagrams of graphical interfaces for reminding a user whether to delay check-in when a skin care application determines that a demonstration action in a skin care check-in plan does not meet a preset implementation condition. As shown in FIG. 8(a), it is assumed that reminder time set by the user for the "Fine line lightening" check-in plan is 15:00. The skin care application determines, by accessing a schedule in the calendar application, that the user is scheduled to hold a meeting at 15:00. In this case, the skin care application prompts, on the graphical interface of the local device, the user whether to delay the fine line lightening check-in. As shown in FIG. 8(a), the user clicks "Yes" to delay the fine line lightening check-in. After the user clicks "Yes" to delay the check-in on the local device, the skin care application may display a graphical interface for time adjustment. Refer to FIG. 8(b). The user may click "Time" 812 in the graphical interface shown in FIG. 8(b) to set time of the next reminder for the fine line lightening check-in. For example, the user sets that the skin care application reminds, at 16:00, the user to perform check-in. Certainly, there may be another intelligent setting. For example, in FIG. 8(a), after the user determines to delay check-in, the skin care application reminds the user whether to perform fine line lightening check-in after a preset time period. For example, the skin care application reminds, every half an hour or every hour, the user whether to perform fine line lightening check-in.

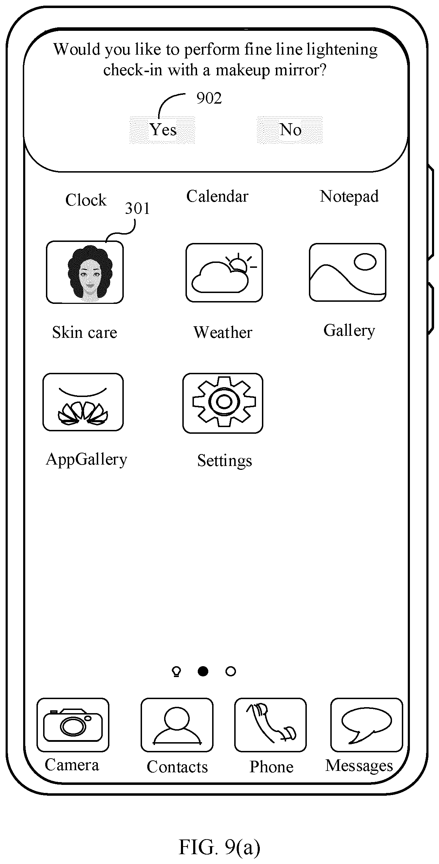

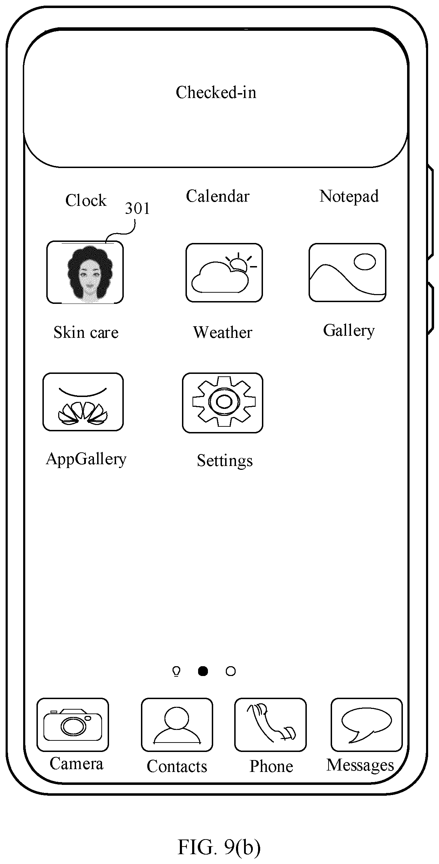

In an example, the skin care application obtains the first target information again one hour later, and determines whether the user meets a preset implementation condition for implementing a fine line lightening check-in plan. It is assumed that it is determined, based on information about a location from which the local device is accessed, that the user is at home, and the makeup mirror detects that the user is in front of the makeup mirror. The makeup mirror may report the information to the skin care application. Refer to FIG. 9(a). The skin care application may remind, on the graphical interface of the local device, the user whether to use the makeup mirror to perform fine line lightening check-in. In this case, the user may click the "Yes" button 902 to determine to capture a skin care action of the user by using the makeup mirror, so as to perform skin care check-in. The makeup mirror may send the captured action to the local device, and the local device may perform matching between the behavior or the action of the user received from the makeup mirror and the demonstration behavior or action for fine line lightening. If the matching is successful, the check-in is completed. Refer to FIG. 9(b). The skin care application prompts, on a graphical interface of the local device, the user to complete the fine line lightening check-in. In this embodiment, the user may not need to start the skin care application on the local device to capture a user action by using a camera of the local device, thereby further improving user experience.

It should be noted that the skin care application may further prompt, by using a voice, the user whether to perform fine line lightening check-in. The user may perform voice interaction with the local device to implement the fine line lightening check-in plan. It may be found that, after the user enables intelligent check-in, the user does not need to enter a check-in page in the skin care application to perform an operation, and the user is intelligently reminded, based on a current status of the user, to determine whether to perform check-in, thereby further improving user experience. To encourage the user to actively complete check-in, the skin care application may further detect whether the user has performed a forbidden behavior, or whether a behavior or an action of the user is qualified relative to the demonstration action. The forbidden behavior indicates a behavior or an action other than the demonstration action in each skin care check-in plan, and specifically indicates a behavior that is not allowed by the user during implementation of the check-in plan. Refer to FIG. 5(i). A forbidden behavior in the fine line lightening check-in plan is "eating spicy food". If it is detected that the user has performed the forbidden behavior, a certain quantity of bonus points may be subtracted as a punishment on the user, and a graphical interface may be further used to remind the user that the forbidden behavior has been performed. If the user has performed a forbidden behavior, it may be considered that the user does not complete the check-in of fine line lightening plan.

The skin care application may determine, in a plurality of manners, whether the user has performed a forbidden behavior. For example, the skin care application determines, by accessing another application, whether the user has performed a forbidden behavior. For example, for the behavior of eating spicy food, the skin care application may access a social application platform used by the user, and determine, according to a food picture and content that are published by the user on the social application platform, whether the user has performed the behavior of eating spicy food. Alternatively, the skin care application may determine, according to information obtained by another device or the local device, whether the user has performed a forbidden behavior. For example, the forbidden behavior is "sleeping late". The skin care application may obtain a time point when the user uses the local device, to determine whether the user sleeps late, or the skin care application may obtain information such as a pulse rate of the user by using a band or another wearable device, to determine whether the user sleeps late.

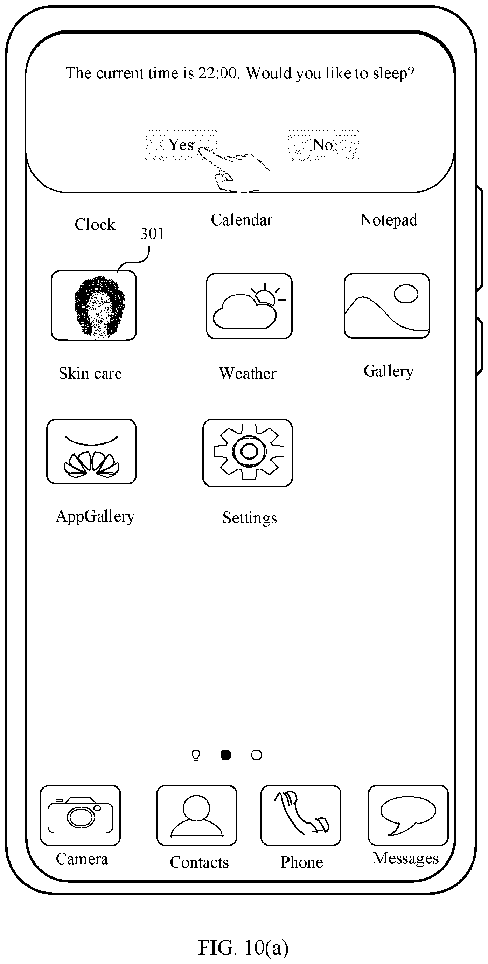

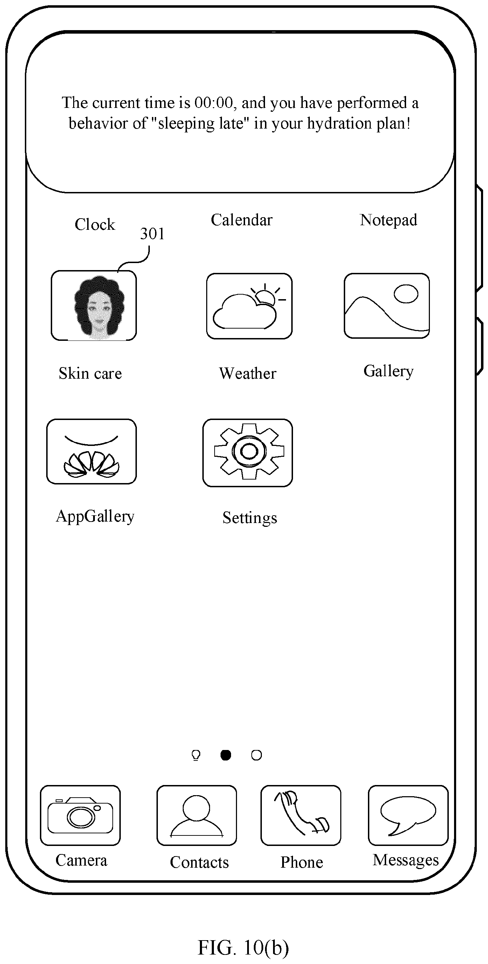

FIG. 10(a) and FIG. 10(b) show graphical interfaces according to an embodiment of this application. It is assumed that a forbidden behavior in a hydration plan added by the user is "sleeping late", and a time point for "sleeping late" is later than 22:00. Refer to FIG. 10(a). When detecting that the current time is 22:00, the skin care application may remind the user to sleep, and the user may click Yes in the figure to start sleeping, so as to complete check-in of the hydration plan. However, the user may continue to do other things without sleeping, that is, the user performs a forbidden behavior in the hydration plan. For example, the skin care application may determine, by accessing a use status of the local device, whether the user has performed a behavior of "sleeping late". If the user still uses the local device after 22:00, the user may be reminded, on a graphical interface of the local device, that the user has performed a corresponding forbidden behavior. Refer to FIG. 10(b). If the skin care application detects that the user still uses the local device at 00:00, the skin care application may remind the user that the user has performed the behavior of "sleeping late", and it is considered that the user does not complete check-in of the hydration plan today.

It should be noted that, when the skin care application detects that the user has performed the forbidden behavior, or when the behavior or the action of the user is not qualified relative to the demonstration action, the user may be reminded on the graphical interface of the mobile phone. The reminding manner includes but is not limited to voice reminding, text reminding shown in FIG. 10(a) and FIG. 10(b), and the like.

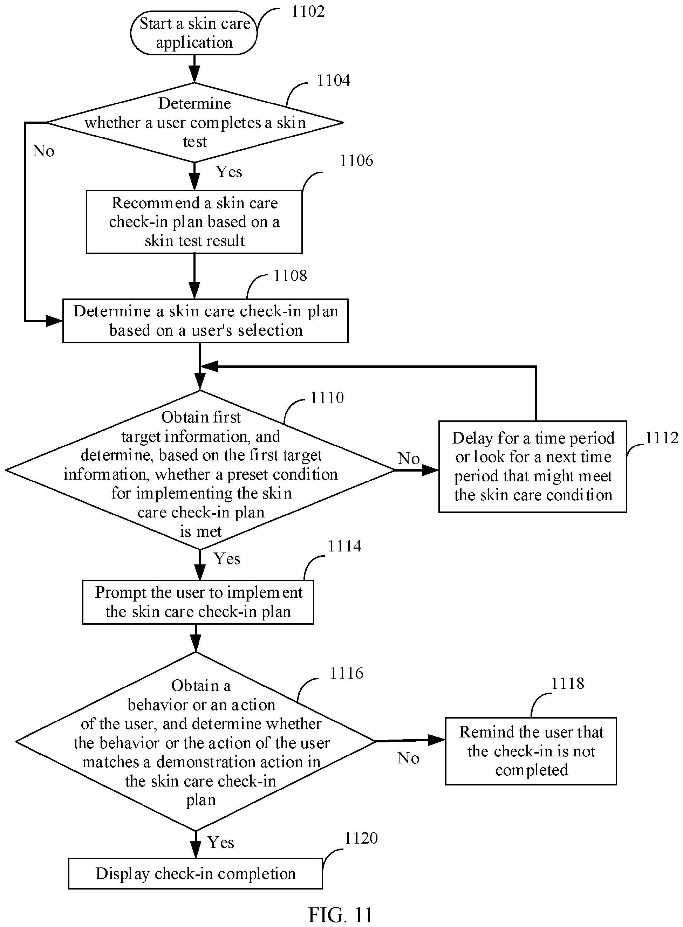

The foregoing has described the skin care check-in method from a perspective of user interaction with reference to the foregoing embodiments and related accompanying drawings. The following describes a skin care check-in method provided in an embodiment of this application from a perspective of a software implementation policy with reference to FIG. 11. It should be understood that the method may be implemented in an electronic device (for example, a mobile phone or a tablet computer) having a hardware structure shown in FIG. 1 and a software system architecture shown in FIG. 2. FIG. 11 is a schematic flowchart of a skin care check-in method implemented by the electronic device according to an embodiment of this application. As shown in FIG. 11, the method may include the following steps.

Step 1102: Open a skin care application.

Refer to FIG. 3(a) to FIG. 3(c). After the skin care application is installed, when the user starts the skin care application for the first time, some precautions for using the skin care application may be displayed. Alternatively, the user can view the precautions on other interfaces of the skin care application when it is not the first time that the user starts the skin care application. The electronic device displays a graphical user interface shown in FIG. 4(a) or FIG. 5(a) in response to the operation of starting the skin care application by the user.

Step 1104: Determine whether the user completes a skin test.

After the skin care application is started, the electronic device detects that it is the first time that user starts the skin care application or that the user does not participate in any skin care check-in plan shown in FIG. 4(a), it is determined that the user has not completed a skin test. The electronic device may prompt, based on a case in which the user does not complete a skin test, the user to perform a skin test. A prompting manner includes a card reminder, dynamic enlarged displaying of the "Skin test" button 406, or the like. Refer to the graphical interface shown in FIG. 4. The user may also click the "Skin test" button 406 without receiving a prompt, to display a skin test interface and obtain a facial image of the user. The skin care application in the local device may analyze the facial image of the user and generate a skin test result. The skin test result may indicate a skin problem of the user.

Specifically, the skin test result of the user can be obtained by using a method such as deep learning or image comparison, and the skin problem of the user can be determined.

Deep learning is used as an example. The skin care application may directly input an obtained image into a trained neural network model, to analyze a skin problem in the image. The following provides a method for training a neural network for analyzing an image, to obtain at least one to-be-analyzed image and a standard analysis image of each to-be-analyzed image, where a category label corresponding to each pixel in each standard analysis image is marked. The category label corresponding to any pixel may be unique identification information corresponding to the pixel. At least one to-be-analyzed image is input into a to-be-trained neural network to obtain at least one training analysis image, and at least one standard analysis image is input into a to-be-trained neural network used for image analysis, where a category label corresponding to each pixel in each training analysis image is marked. The to-be-trained neural network used for image analysis includes a convolution part, a deconvolution part, and an output part. The convolution part includes at least one layer of convolutional neural network. Each layer of convolutional neural network includes a convolution layer, an activation layer, and a pooling layer. The deconvolution part includes at least one layer of deconvolutional neural network. Each layer of deconvolutional neural network includes a deconvolution layer and a convolution layer. The output part includes a convolution layer and an output layer, where the output layer outputs a result by using a classifier. For each training analysis image, a category label of each pixel of the training analysis image is obtained; and for each standard analysis image, a category label of each pixel of the standard analysis image is obtained. For each training analysis image, a neural network to be trained for image analysis is trained, based on a category label difference between an image feature of the training analysis image and each pixel of a standard analysis image of the training analysis image, to obtain a target neural network used for image analysis. Specifically, a value of a loss function of the neural network to be trained for image analysis may be obtained based on a category label difference between each pixel of the training analysis image and each pixel of the standard analysis image of the training analysis image, and a model parameter of the neural network to be trained for image analysis is updated based on the value of the loss function. Generally, a smaller value of the loss function indicates a more accurate output result of the neural network. The value of the loss function obtained each time may be compared with a preset value. If an absolute value of a difference between the two values is less than a preset standard value, the model parameter of the neural network to be trained for image analysis is updated based on the value of the loss function. It should be noted that this embodiment shows only a method for training a neural network used for image analysis, and there is another training method. Details are not described herein.

For example, the skin care application analyzes the skin of the user through image comparison. For example, after obtaining an image of an area such as a face of the user, the skin care application first pre-processes the image by using a mobile phone, to remove image noise and an irrelevant background image, so as to obtain a binarized image. Then Euclidean distance transformation is performed on the binarized image to obtain a highlight line of the Euclidean distance transformation, vertical analysis is performed on the image obtained after the Euclidean distance transformation, so as to extract a texture feature of a region of interest, the extracted texture feature is compared with a texture feature of a skin image having a related skin problem, and then the image is recognized or classified by using a classifier, so as to obtain a skin test result of the user, and determine the skin problem of the user.

It may be understood that obtaining the skin test result of the user through analysis is not limited to being completed on the local device, and another computing device may be used for analysis, and the skin test result obtained through analysis is sent back to the local device.

After the user completes the skin test, step 1106 is performed.

Step 1106: The skin care application recommends a skin care check-in plan based on a skin test result of the user.

Specifically, the skin care application displays, on a display interface of the local device based on the skin test result, a skin care check-in plan recommended to the user. For example, if the skin of the user has dehydration problem, the skin care application recommends a hydration check-in plan to the user. Alternatively, if the skin of the user has fine lines, a fine line lightening check-in plan may be recommended to the user.

Step 1108: Determine a skin care check-in plan based on the user's selection.

After the skin care application recommends the skin care check-in plan to the user, step 1108 is performed. It should be noted that the user may also join, on another page of the skin care application, a skin care check-in plan as required without performing the foregoing steps 1104 and 1106. For example, the user can directly add the skin care check-in plan on the graphical interfaces shown in FIG. 5(a) and FIG. 5(b). After the user adds the skin care check-in plan, the electronic device determines, based on the user's selection, the skin care check-in plan to be implemented by the user.

Step 1110: Obtain the first target information, and determine, based on the first target information, whether the user meets a preset implementation condition for implementing the skin care check-in plan.

The preset implementation condition is preset by the skin care application and is used to indicate that the user currently meets a condition for implementing a skin care check-in plan to which the user joins. Each skin care check-in plan to which the user joins has a specific preset implementation condition. The specific preset implementation condition includes an implementation condition required for each demonstration action in the skin care check-in plan. The preset implementation condition is determined according to the first target information obtained by the skin care application.

The first target information includes but is not limited to movement status information of the user, device location information of the user, calendar schedule information of the user, biological feature information of the user, life behavior information of the user, and the like. The first target information may be obtained by using a device such as a local device, a wearable device, a smart home device, or an accessory.

In an example, after the user joins the skin care check-in plan, information such as a movement status of the user is detected by using the local device, a wearable device connected to the local device, a smart home device, a peripheral, a mobile phone, or an in-vehicle device. For example, a device such as the local device may obtain an amplitude, a frequency, and the like of an arm movement of the user by using an acceleration sensor, to determine whether the user is currently in a movement state, or obtain body feature information such as a heart rate of the user by using a heart rate sensor built in another peripheral. It may be understood that, if the amplitude and frequency of the arm movement of the user are excessively high and the heart rate of the user is relatively high, it may be determined that the user is doing exercise. If the user is in the movement state, the skin care application may determine that it is not suitable for the user to implement or the user cannot implement the skin care check-in plan, that is, the user does not meet the preset implementation condition for implementing the skin care check-in plan. However, if it is detected that the heartbeat of the user is smooth and the body of the user is in a relatively quiet state, the user may be prompted to perform skin care check-in.

In an example, after the user joins the skin care check-in plan, the user interacts with a head unit in a transportation means such as a vehicle through the local device, to determine whether the user is driving the vehicle. If the user is driving the vehicle, it may be determined that it is not suitable for the user to implement the skin care check-in plan, that is, the user does not meet the condition for implementing the skin care check-in plan.

In an example, the skin care application may access an application installed on the local device to obtain the first target information of the user. For example, the skin care application may access a calendar schedule on the local device, and may determine, by accessing the calendar schedule, that the user can implement the skin care check-in plan. For example, if it is determined, by accessing a calendar schedule, that the user is currently in a meeting, it is not suitable for the user to implement the skin care check-in plan, that is, the skin care application may not remind the user to perform skin care check-in, or may ask the user whether to delay skin care check-in. When the skin care application determines, by accessing the current calendar schedule of the user, that the user is not currently scheduled for a task, the skin care application may prompt the user to implement the skin care check-in plan.

In an example, whether the user is at home and a current status of the user may be identified by using a smart household appliance. For example, the user may be doing housekeeping in a kitchen or taking a rest in a living room. If the user is currently doing housekeeping, the skin care application may determine that it is not suitable for the user to implement the skin care check-in plan. If the user is taking a rest in the living room, the skin care application may determine that it is suitable for the user to implement the skin care check-in plan, and may remind the user to implement the skin care check-in plan.

In an example, the skin care application may access location information and time information of the local device to determine whether it is suitable for the user to implement the skin care check-in plan. For example, if the current time is 10:00 a.m. on a working day, and the location of the user is the user's office, it may be not suitable for the user to implement the skin care check-in plan, and the skin care application may not remind the user to implement the skin care check-in plan. It should be noted that the location information includes but is not limited to GPS or Bluetooth location information.

It may be understood that the skin care application may make a decision based on more first target information, to determine whether the user is currently suitable for implementing the skin care check-in plan. For example, the skin care application detects, at a first moment, that the user is outdoors and is in the movement state, and does not remind the user to implement the skin care check-in plan. At a second moment, when it is detected that the user's body is in a relatively static state, the heart rate is smooth, the current location of the device is the user's home, and it is determined, by accessing a calendar schedule application, that the user has no schedule for a next preset time period, it is determined that the current user status is suitable for implementing the skin care check-in plan.

In an embodiment, when the skin care application determines, based on the first target information, that the user does not meet the condition for implementing the skin care check-in plan, the skin care application may automatically re-obtain the first target information of the user in one or more preset time periods to determine whether the user meets the preset implementation condition for implementing the skin care check-in plan (refer to step 1112). When the user meets the condition for implementing the skin care check-in plan, the skin care check-in application reminds the user to implement the skin care check-in plan.

Alternatively, when determining, based on the first target information, that the user does not meet the condition for implementing the skin care check-in plan, the skin care application may prompt, based on time set by the user, the user whether to delay check-in for a period of time.

In an embodiment, when the user meets the preset implementation condition for implementing the skin care check-in plan, step 1114 is performed.

Step 1114: Prompt the user to implement the skin care check-in plan.

When the skin care application determines, based on the first target information, that the user meets the preset implementation condition for implementing the skin care check-in plan, the skin care application may prompt the user to implement the skin care check-in plan. FIG. 9(a) and FIG. 10(a) show examples of graphical interfaces used for reminding a user to implement a skin care check-in plan.

It may be understood that more reminding manners may be set. This is not limited herein.

Step 1116: Obtain a behavior or an action of the user, and determine whether the behavior or the action of the user matches a demonstration action in the skin care check-in plan.

In an embodiment, when the user determines to implement the skin care check-in plan, the skin care application obtains and identifies the behavior or the action of the user by using a device such as the local device, a wearable device, a makeup mirror, a smart screen, or a mobile phone. Specifically, the skin care application may identify a gesture posture, a gesture position, step duration of the skin care action, and an amplitude of the action of the user, and then perform matching between the behavior or the action of the user and a demonstration action in the skin care check-in plan. It should be noted that, in an identification and matching process, matching and identification may be performed by using a technical means such as deep learning or pattern identification. This is not limited herein.

For example, refer to FIG. 5(j). After the user clicks the "Start" button, a corresponding skin care action starts to be implemented. In addition, the skin care application invokes a camera of the local device or a camera of another device (for example, a makeup mirror) connected to the local device, to detect and obtain a behavior or an action of the user, so as to perform matching.

It should be noted that the manner in which the skin care application invokes the camera to detect and obtain the behavior and the action of the user is not limited to triggering by clicking the "Start" button on the interface shown in FIG. 5(j), and may be triggering on another interface in another manner. For example, on the interface shown in FIG. 9(a), after the user clicks "Yes", the skin care application is directly triggered to invoke the camera, and the user does not need to complete the operation on the graphical interface shown in FIG. 5(j). Alternatively, the user may enter an instruction in a voice manner, to trigger the skin care application to invoke the camera. Specifically, when the skin care application determines, based on the first target information, that the skin care check-in plan meets the preset implementation condition, the skin care application may prompt, by using a voice, the user whether to implement the skin care mode, and the user may input an instruction for implementing the skin care mode to the skin care application in a voice input manner. For example, the skin care application may perform matching between each behavior or action of the user and a demonstration action in a skin care check-in plan. If the behavior or the action of the user matches the demonstration action, a quantity of qualified actions is accumulated; or if a total quantity of qualified actions meets a preset quantity, check-in completion is displayed; or if a total quantity of qualified actions does not meet the preset quantity, check-in completion is not displayed. For example, a check-in plan includes 10 action combinations. If more than six behaviors or actions of the user match actions in the action combination, it is considered that the user completes the demonstration action; or if fewer than five behaviors or actions of the user match the actions in the action combination, it is considered that the user does not complete the demonstration action, and check-in completion by the user is not displayed, or the user is prompted that the user's action is not standard or incorrect (step 1118).

In a non-limiting embodiment, a difference value between a force of each behavior or action of the user and a force of each demonstration action may be further compared. If the difference value is less than a preset threshold, it is considered that each behavior or action of the user matches the demonstration action. Specifically, the force of the action of the user may be detected by using an acceleration sensor on a wearable device. Certainly, a matching degree between duration of each behavior or action of the user and duration of each demonstration action may be further determined by detecting the duration of the action of the user. If face washing duration of the user exceeds preset duration specified in the skin care check-in plan, it is determined that a matching degree between the behavior or the action of the user and a specific behavior or action of the skin care mode is relatively small, that is, the action or the behavior of the user is not qualified.

In an example, if the behavior or the action of the user matches a demonstration action in the skin care check-in plan, step 1120 is performed.

Step 1120: Display check-in completion.

In an example, if the behavior or the action of the user matches any demonstration action in the skin care check-in plan, the skin care application display completion of check-in of the skin care mode.

Refer to the interfaces shown in FIG. 5(l) or FIG. 9(b). For example, the skin care application displays completion of check-in of the skin care mode.

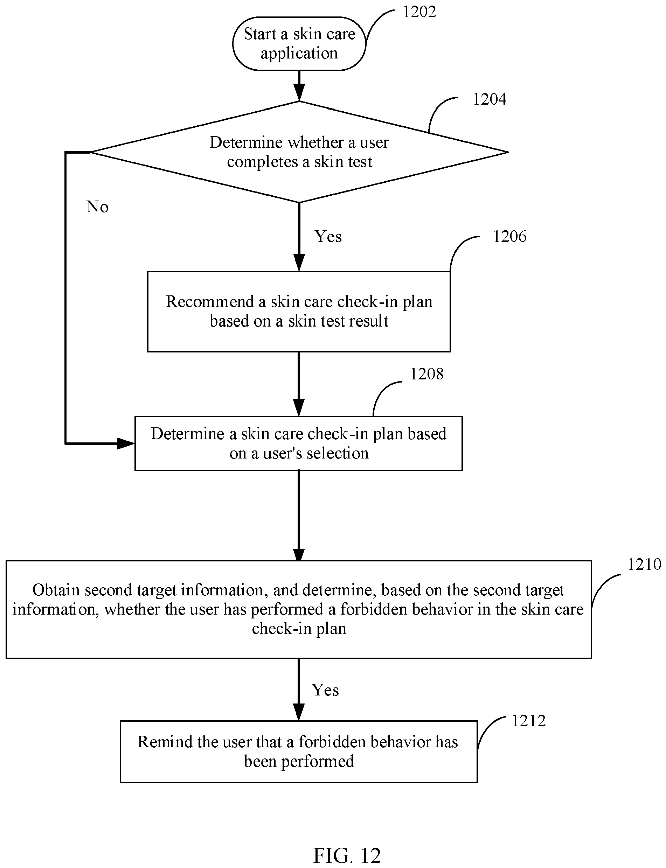

The following describes a skin care check-in method provided in another embodiment of this application from a perspective of a software implementation policy with reference to FIG. 12. Steps 1202 to 1208 are the same as steps 1102 to 1108, and details are not described herein again. Step 1210: Obtain second target information, and determine, based on the second target information, whether the user has performed a forbidden behavior in the skin care check-in plan.

The second target information includes but is not limited to biological feature information of the user, life behavior information of the user, a location of an electronic device, use time, content of an application, and the like. The second target information may be obtained by using a device such as a local device, a wearable device, a smart home device, or an accessory. The skin care application may determine, based on the second target information, whether the application has performed a forbidden behavior in the skin care check-in plan.

The forbidden behavior may include a behavior that is not allowed in each skin care solution other than a demonstration action, and may further include a non-standard behavior performed by the user when the user performs a demonstration action, for example, an action of the user is not qualified, or duration of a behavior or an action of the user exceeds duration specified in a demonstration action.

The skin care application may determine, in a plurality of manners, whether the user has performed a forbidden behavior. For example, the skin care application determines, by accessing another application, whether the user has performed a forbidden behavior. For example, for the behavior of eating spicy food, the skin care application may access a social application platform used by the user, and determine, according to a food picture and content that are published by the user on the social application platform, whether the user has performed the behavior of eating spicy food. Alternatively, the skin care application may determine, according to information obtained by another device or the local device, whether the user has performed a forbidden behavior. For example, the forbidden behavior is "sleeping late". The skin care application may obtain time when the user uses the local device, to determine whether the user sleeps late, or the skin care application may obtain information such as a pulse rate of the user by using a band or another wearable device, to determine whether the user sleeps late. Alternatively, the skin care application may further determine, by using the wearable device, whether a face washing force of the user meets a requirement of the behavior, or the like.

Step 1212: Remind the user that a forbidden behavior has been performed.

When it is detected that the user has performed a forbidden behavior, the skin care application may remind, on a graphical interface, the user that the forbidden behavior has been performed. FIG. 10(b) shows a graphical interface on which the skin care application reminds the user that the forbidden behavior has been performed.

It may be understood that the bonus points obtained by the user through skin care check-in may be further subtracted, or skin care check-in completed by the user on a current day may be canceled, so as to achieve an effect of monitoring and encouraging the user. According to the skin care check-in method provided in this application, the user does not need to manually check in each time the user implements a skin care check-in plan, and the skin care application may prompt the user to perform check-in when determining, based on the current status of the user, that a skin care solution in the skin care plan meets a preset implementation condition. This reduces the workload of the user while supervising the user, thereby improving user experience.

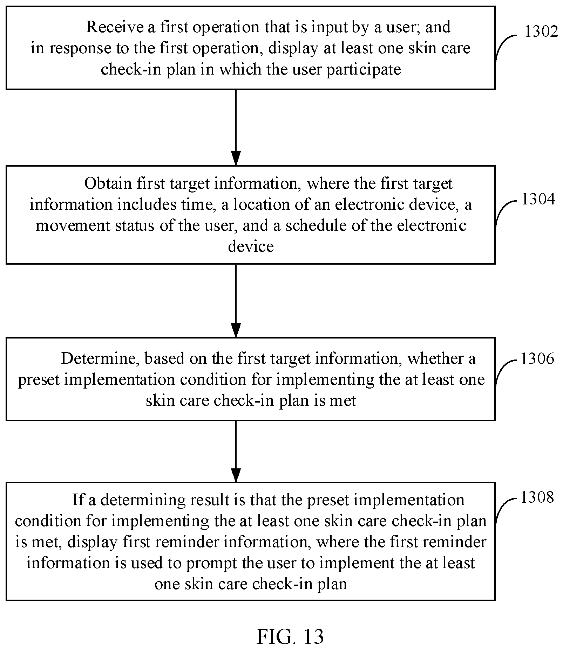

FIG. 13 is a flowchart of a skin care check-in method according to another embodiment of this application. The method includes:
Step 1302: An electronic device receives a first operation that is input by a user; and
   in response to the first operation, displays at least one skin care check-in plan in which the user participates.
Step 1304: The electronic device obtains first target information, where the first target information includes time, a location of the electronic device, a movement status of the user, and a schedule of the electronic device.
Step 1306: The electronic device determines, based on the first target information, whether a preset implementation condition for implementing the at least one skin care check-in plan is met.

The preset implementation condition is preset by the skin care application and is used to indicate that the user currently meets a condition for implementing a skin care check-in plan to which the user joins. Each skin care check-in plan to which the user joins has a specific preset implementation condition. The specific preset implementation condition includes an implementation condition required for each demonstration action in the skin care check-in plan. The preset implementation condition is determined according to the first target information obtained by the skin care application.

Step 1308: If a determining result is that the preset implementation condition for implementing the at least one skin care check-in plan is met, the electronic device displays first reminder information, where the first reminder information is used to prompt the user to implement the at least one skin care check-in plan.

An embodiment of this application further provides a computer-readable storage medium, where the computer-readable storage medium includes computer instructions, and when the computer instructions are run on an electronic device, the electronic device is enabled to perform the method in FIG. 13 of this application.

Functional units in this embodiment of this application may be integrated into one processing unit, or each of the units may exist alone physically, or two or more units are integrated into one unit. The integrated unit may be implemented in a form of hardware, or may be implemented in a form of a software functional unit.

When the integrated unit is implemented in the form of a software functional unit and sold or used as an independent product, the integrated unit may be stored in a computer-readable storage medium. Based on such an understanding, the technical solutions of embodiments of this application essentially, or the part contributing to the conventional technology, or all or some of the technical solutions may be implemented in the form of a software product. The computer software product is stored in a storage medium and includes several instructions for instructing a computer device (which may be a personal computer, a server, or a network device) or a processor to perform all or some of the steps of the methods described in embodiments of this application. The foregoing storage medium includes any medium that can store program code, such as a flash memory, a removable hard disk, a read-only memory, a random access memory, a magnetic disk, or an optical disc.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A skin care check-in method, applied to an electronic device, comprising:
receiving a first operation that is input by a user;
in response to the first operation, displaying at least one skin care check-in plan in which the user participates;
obtaining first target information, wherein the first target information comprises one or more of time, a location of the electronic device, a movement status of the user, and a schedule of the electronic device;
determining, based on the first target information, whether a preset implementation condition for implementing the at least one skin care check-in plan is met; and
if a determining result is that the preset implementation condition for implementing the at least one skin care check-in plan is met, displaying first reminder information, wherein the first reminder information is used to prompt the user to implement the at least one skin care check-in plan.

2. The check-in method according to claim 1, wherein the method further comprises:
receiving a second operation that is input by the user;
in response to the second operation, obtaining at least one behavior or action of the user;
determining whether the at least one behavior or action matches a corresponding demonstration action, wherein the corresponding demonstration action is a demonstration action in the at least one skin care check-in plan; and
if a determining result is that the at least one behavior or action matches the corresponding demonstration action, displaying second reminder information, wherein the second reminder information is used to prompt that the at least one skin care check-in plan is completed; or
if a determining result is that the at least one behavior or action does not match the corresponding demonstration action, displaying third reminder information, wherein the third reminder information is used to prompt that the at least one skin care check-in plan is not completed.

3. The check-in method according to claim 1, wherein the method further comprises:
if a determining result is that the preset implementation condition for implementing the at least one skin care check-in plan is not met, displaying fourth reminder information, wherein the fourth reminder information is used to prompt the user to determine whether to delay implementation of the at least one skin care check-in plan.

4. The check-in method according to claim 3, wherein the method further comprises:
receiving a third operation that is input by the user; and
in response to the third operation, re-obtaining the first target information after predetermined time, and determining, based on the re-obtained first target information, whether the preset implementation condition for implementing the at least one skin care check-in plan is met.

5. The check-in method according to claim 1, wherein the method further comprises:
if a determining result is that the preset implementation condition is not met, re-obtaining the first target information after predetermined time, and determining, based on the re-obtained first target information, whether the preset implementation condition for implementing the at least one skin care check-in plan is met.

6. The check-in method according to claim 1, wherein the method further comprises:
obtaining second target information, wherein the second target information comprises at least one of time, a location of the electronic device, and content of an application;
determining, based on the second target information, whether the user has performed a forbidden behavior in the at least one skin care check-in plan; and
if a determining result is that the forbidden behavior is implemented, displaying fifth reminder information, wherein the fifth reminder information is used to remind the user that the forbidden behavior is implemented or the skin care check-in plan is not completed.

7. The check-in method according to claim 1, wherein the method further comprises:
receiving a fourth operation that is input by the user;
in response to the fourth operation, obtaining a facial image of the user;
obtaining a facial image analysis result based on the facial image;
displaying at least one skin care check-in plan based on the facial image analysis result;
receiving a fifth operation that is input by the user, wherein the fifth operation is used to select a skin care check-in plan in which the user does not participate; and
in response to the received fifth operation, adding the skin care check-in plan.

8. An electronic device, comprising a processor and a storage device, wherein the storage device stores an application, and when the application is run by the processor, the electronic device is enabled to perform the method according to any one of claims 1 to 7.

9. A computer-readable storage medium, comprising computer instructions, wherein when the computer instructions are run on an electronic device, the electronic device is enabled to perform the method according to any one of claims 1 to 7.

10. A computer program product, wherein when the computer program product runs on a computer, the computer is enabled to perform the method according to any one of claims 1 to 7.
